# EUROPEAN PATENT SPECIFICATION

(11) **EP 4 097 247 B1**
(45) Date of publication and mention of the grant of the patent: **01.10.2025**
(21) Application number: 21748177.9
(22) Date of filing: 28.01.2021
(51) Int. Cl.: C12Q 1/68, C12Q 1/6816, C12Q 1/6827, C12Q 1/6832, C12Q 1/6834, C12Q 1/6876, C12Q 1/682, G01N 33/542, G01N 33/557, G01N 33/543

(54) **ANALYTE DETECTION**
ANALYTNACHWEIS
DÉTECTION D'ANALYTE

(30) Priority: 29.01.2020 US 202062967433 P
(43) Date of publication of application: 07.12.2022
(73) Proprietor: The Regents of The University of Michigan, Ann Arbor, Michigan 48109-2590 (US)
(72) Inventor: JOHNSON-BUCK, Alexander, Ann Arbor, Michigan 48109-2590 (US)
(74) Representative: Glawe, Delfs, Moll
(86) International application number: PCT/US2021/015407
(87) International publication number: WO 2021/154935

(56) References cited:
- WO-A1-2020/023503
- WO-A2-2006/128138
- US-A1- 2010 158 850
- US-A1- 2016 054 261
- US-A1- 2018 002 743
- US-A1- 2018 258 469
- US-A1- 2019 187 031
- MONTSERRAT SHELBOURNE ET AL: "Fast copper-free click DNA ligation by the ring-strain promoted alkyne-azide cycloaddition reaction", CHEMICAL COMMUNICATIONS, vol. 47, no. 22, 1 January 2011 (2011-01-01), pages 6257, XP055057365, ISSN: 1359-7345, DOI: 10.1039/c1cc10743g
- AFAF H. EL-SAGHEER ET AL: "Click Nucleic Acid Ligation: Applications in Biology and Nanotechnology", ACCOUNTS OF CHEMICAL RESEARCH, vol. 45, no. 8, 21 August 2012 (2012-08-21), US, pages 1258 - 1267, XP055454304, ISSN: 0001-4842, DOI: 10.1021/ar200321n
- CHATTERJEE TANMAY, KNAPPIK ACHIM, SANDFORD ERIN, TEWARI MUNEESH, CHOI SUNG WON, STRONG WILLIAM B, THRUSH EVAN P, OH KENNETH J, LIU: "Direct kinetic fingerprinting and digital counting of single protein molecules", PROC. NATL. ACAD. SCI. U.S.A, vol. 117, no. 37, 31 August 2020 (2020-08-31), pages 22815 - 22822, XP055846344

## Description

This application claims priority to United States provisional patent application serial number 62/967,433, filed January 29, 2020.

### FIELD

Provided herein is technology relating to analyte detection and particularly, but not exclusively, to compositions, methods, systems, and kits for detecting, characterizing, identifying, and/or quantifying analytes (e.g., nucleic acids, proteins, or other biomolecules) using molecular probes that integrate cumulative repeated binding of probes to the same analyte molecule.

### BACKGROUND

The sensitive and specific detection of analytes (e.g., including, but not limited to, biomolecules (e.g., proteins, nucleic acids, and lipids) and small molecules (e.g., drugs, metabolites, cofactors, etc.)) plays an important role in biomedical research, in vitro diagnostics, and clinical medicine. Analyte detection is generally carried out using molecular reagents (e.g., antibodies, hybridization probes, etc.) that have a high affinity and specificity for the analyte of interest and that produce a detectable signal only in the presence of the target analyte. However, affinity probes have a finite specificity and affinity for the target analyte (see, e.g., Zhang et al. (2012) "Optimizing the specificity of nucleic acid hybridization. Nat. Chem. 4, 208-14), thus making it challenging to detect some analytes with high confidence. This is a significant problem for biomarkers of interest that are present at vanishingly small concentrations (e.g., at sub-femtomolar concentrations; see, e.g., Lee et al. (2001) "Quantitation of genomic DNA in plasma and serum samples: higher concentrations of genomic DNA found in serum than in plasma." Transfusion (Paris) 41: 276-82; Mitchell et al. (2008) "Circulating microRNAs as stable blood-based markers for cancer detection" Proc. Natl. Acad. Sci. U. S. A. 105: 10513-18; Bettegowda et al. (2014) "Detection of Circulating Tumor DNA in Early- and Late-Stage Human Malignancies" Sci. Transl. Med. 6: 224ra24-224ra24; Husain et al. (2017) "Monitoring Daily Dynamics of Early Tumor Response to Targeted Therapy by Detecting Circulating Tumor DNA in Urine" Clin. Cancer Res. 23: 4716-23).

While sensitivity can be increased by producing many copies of the analyte (e.g., by PCR and other nucleic acid amplification technologies), amplifying the signal, or using long assay and observation times to record a signal, analyte detection technologies would be improved by providing an end-point detection technology with a decreased limit of detection.

Compositions for detecting an analyte in a sample are known from the prior art, e.g. MONTSERRAT SHELBOURNE ET AL: "Fast copper-free click DNA ligation by the ring-strain promoted alkyne-azide cycloaddition reaction", CHEMICAL COMMUNICATIONS, vol. 47, no. 22, 1 January 2011, page 6257; AFAF H. EL-SAGHEER ET AL: "Click Nucleic Acid Ligation: Applications in Biology and Nanotechnology", ACCOUNTS OF CHEMICAL RESEARCH, vol. 45, no. 8, 21 August 2012, pages 1258-1267; WO 2006/128138 A2; US 2018/002743 A1; and US 2019/187031 A1. None of said prior art documents disclose or suggest the subject-matter of the present invention as defined in the appended claims.

### SUMMARY

The invention is set out in the appended claims. Hereinafter embodiments are embodiments of the invention only, if they fall within the scope of the claims. Otherwise they are mere embodiments of the broader disclosure. Accordingly, provided herein is a technology for detecting an analyte using molecular probes that integrate cumulative repeated binding of probes to the same analyte molecule. The technology is similar to Single-Molecule Recognition with Equilibrium Poisson Sampling (SiMREPS). See, e.g., U.S. Pat. App. Ser. No. 14/589,467; and Johnson-Buck et al. (2016) "Kinetic fingerprinting to identify and count single nucleic acids" Nature Biotechnology 33: 790.

However, the technology described herein differs from SiMREPS in that the repeated binding of one probe (e.g., a "tally" probe as described herein, which is similar to a SiMREPS "query" probe) results in the accumulation of multiple labels on another probe ("integrator" probe) that records the repeated binding. The presence of the analyte is indicated by the presence of one or more integrator probes comprising multiple (e.g., more than one) labels attached to it. Accordingly, the repeated binding of tally probes to the analyte is not necessarily observed in real time in the present technology, but is detected (e.g., using an end-point measurement) by the number of labels attached to each integrator probe, e.g., either at the single-molecule level (e.g., using fluorescence microscopy) or using bulk methods (e.g., electrophoresis, chromatography, or mass spectrometry).

The technology disclosed herein provides for improved (e.g., accelerated) and simpler data acquisition and/or data analysis because said technology can acquire data using end-point measurements rather than as a time-dependent real-time signal. However, the technology is not limited to using end-point detection and may comprise use of real-time measurements, e.g., to record a signal and/or a time-dependent signal. The present technology disclosed herein provides a lower limit of detection than some existing (e.g., real-time (e.g., SiMREPS)) technologies, e.g., by detecting a much larger fraction of the analyte in a sample relative to existing (e.g., real-time (e.g., SiMREPS)) technologies. The technology described herein finds use in, e.g., diagnostics, research, and clinical medicine and may increase the accuracy of molecular recognition in targeted therapeutics.

Accordingly, provided herein are compositions for detecting a nucleic acid analyte in a sample as defined in the claims. The technology described herein provides a composition comprising an integrator probe specific for an analyte; and a tally probe specific for the analyte and comprising a label, wherein the label is irreversibly (e.g., substantially or effectively irreversibly) transferred from the tally probe to the integrator probe when the integrator probe and the tally probe are both bound to the analyte. The integrator probe may stably bind the analyte. The tally probe may transiently bind the analyte. The integrator probe may be configured to bind more than one label (e.g., 2, 3, 4, 5, 6, 7, 8, 9, 10, 11, 12, 13, 14, 15, 16, 17, 18, 19, or 20, or more labels). A composition may comprise an integrator probe comprising multiple labels (e.g., 2, 3, 4, 5, 6, 7, 8, 9, 10, 11, 12, 13, 14, 15, 16, 17, 18, 19, or 20, or more labels). Compositions as described herein may further comprise an analyte. The integrator probe may be configured to bind a label non-covalently (e.g., irreversibly, substantially irreversibly, and/or effectively irreversibly non-covalently). The integrator probe may be configured to bind a label covalently (e.g., irreversibly, substantially irreversibly, and/or effectively irreversibly covalently). The analyte of the broader disclosure may comprise a nucleic acid, a protein, a metabolite, a small molecule, a lipid, or a sugar. In the invention the analyte is a nucleic acid. In the broader disclosure the integrator probe may comprise a nucleic acid or a protein (e.g., an antibody) or both a nucleic acid and a protein; in the invention it comprises a nucleic acid. In the broader disclosure the tally probe may comprise a nucleic acid or a protein (e.g., an antibody) or both a nucleic acid and a protein; in the invention it is nucleic acid based and comprises a region of RNA nucleotides targeted for RNAse degradation. The label may be a fluorescent or luminescent label. The composition may further comprise a tally probe release component that converts a tally probe bound to the analyte to a dissociated tally probe. The tally probe release component may comprise an enzyme specific for a tally probe-analyte complex in the broader disclosure; in the invention this is an RNAse. The integrator probe may comprise a first functional group and the label of the tally probe may comprise a second functional group that is selectively reactive towards the first functional group. The integrator probe may comprise a first click reactant and the label of the tally probe may comprise a second click reactant; in the invention these are complementary click chemistry groups. The integrator probe may comprise a first affinity ligand and the label of the tally probe may comprise a second affinity ligand that selectively binds the first affinity ligand. The integrator probe may comprise a first nucleic acid sequence and the label of the tally probe may comprise a second nucleic acid sequence complementary to the first nucleic acid sequence. The transfer of the label from the tally probe to the integrator probe may occur via toehold-mediated strand displacement. The integrator probe may comprise a consensus target amino acid or nucleic acid sequence, and the label of the tally probe may comprise a ligand that is enzymatically transferred to the consensus target sequence. The integrator probe may comprise a separate solid or liquid phase (e.g., a colloidal particle, droplet of a liquid-in-liquid emulsion, or phase of an aqueous two-phase system) into which the label of the tally probe is selectively partitioned upon binding of the tally probe to the analyte.

In a further example, the technology provides a composition comprising an integrator probe that is capable of stably associating with an analyte; and a tally probe comprising a label and that is capable of directly or indirectly associating with the analyte, wherein irreversible transfer of the label from the tally probe to the integrator probe occurs more rapidly and/or more efficiently when the tally probe and the integrator probe are both associated with the analyte than when the tally probe and/or the integrator probe is/are dissociated from the analyte. The composition may further comprise an adaptor probe that is capable of binding to the analyte and wherein the tally probe indirectly associates with the analyte by directly associating with the adaptor probe. The tally probe may be specific for the analyte and may directly associate with the analyte. The integrator probe may be specific for the analyte. The tally probe may transiently bind the analyte. The tally probe may transiently bind the adaptor probe. The integrator probe may be configured to bind more than one label. The composition may comprise an integrator probe comprising multiple labels. The composition may further comprise an analyte. The integrator probe may be configured to bind a label non-covalently. The integrator probe may be configured to bind a label covalently. The analyte may comprise a nucleic acid, a protein, a metabolite, a small molecule, a lipid, or a sugar. The integrator probe may comprise a separate phase of matter. The separate phase of matter may be a colloidal particle, liposome, micelle, or emulsified droplet. For example, a droplet or particle may comprise the analyte (e.g., the analyte is trapped on/inside a droplet or particle) and the binding of the tally probe to the analyte may result in transfer of a label to the droplet or particle. The integrator probe may comprise a nucleic acid or a protein. The tally probe may comprise a nucleic acid or a protein. The label may be a fluorescent or luminescent label. The composition may further comprise a tally probe release component that converts a tally probe bound to the analyte to a dissociated tally probe. The tally probe release component may comprise an enzyme specific for a tally probe-analyte complex. The integrator probe may comprise a first functional group and the label of the tally probe may comprise a second functional group that is reactive with the first functional group. The integrator probe may comprise a first click reactant and the label of the tally probe may comprise a second click reactant.

Furthermore, the technology provides a system for detecting an analyte in a sample. For example, systems comprise an integrator probe specific for an analyte; and a tally probe specific for the analyte and comprising a label, wherein the label is irreversibly (e.g., substantially or effectively irreversibly) transferred from the tally probe to the integrator probe when the integrator probe and the tally probe are both bound to the analyte. The integrator probe may stably bind the analyte. The tally probe may transiently bind the analyte. The integrator probe may be configured to bind more than one label (e.g., 2, 3, 4, 5, 6, 7, 8, 9, 10, 11, 12, 13, 14, 15, 16, 17, 18, 19, or 20, or more labels). Systems may further comprise an analyte. The analyte may comprises a nucleic acid, a protein, a metabolite, a small molecule, a lipid, or a sugar. The integrator probe may comprise a nucleic acid or a protein (e.g., an antibody) or both a nucleic acid and a protein. The tally probe may comprise a nucleic acid or a protein (e.g., an antibody) or both a nucleic acid and a protein. The label may be a fluorescent or luminescent label. Systems may further comprise a detection component configured to detect and/or quantify a signal (e.g., a fluorescence signal, a mass signal, a charge signal) produced by an integrator probe comprising multiple labels. Systems may comprise a microprocessor (e.g., a computer). Systems may comprise a software component configured to record a signal produced by an integrator probe, configured to detect and/or quantify the presence of an analyte in a sample using a signal produced by an integrator probe, and/or to produce an output comprising a qualitative and/or quantitative value describing the presence and/or quantity (e.g., amount and/or concentration) of an analyte in a sample. The integrator probe may comprise a first functional group and the label of the tally probe may comprise a second functional group that is selectively reactive towards the first functional group. The integrator probe may comprise a first click reactant and the label of the tally probe may comprise a second click reactant. The integrator probe may comprise a first affinity ligand and the label of the tally probe may comprise a second affinity ligand that selectively binds the first affinity ligand. The integrator probe may comprise a first nucleic acid sequence and the label of the tally probe may comprise a second nucleic acid sequence complementary to the first nucleic acid sequence. The transfer of the label from the tally probe to the integrator probe may occur via toehold-mediated strand displacement. The integrator probe may comprise a consensus target amino acid or nucleic acid sequence and the label of the tally probe may comprise a ligand that is enzymatically transferred to the consensus target sequence. Systems may further comprise a tally probe release component that converts a tally probe bound to the analyte to a dissociated tally probe. The tally probe release component may comprise an enzyme specific for a tally probe-analyte complex. The integrator probe may comprise a separate solid or liquid phase (e.g., a colloidal particle, droplet of a liquid-in-liquid emulsion, or phase of an aqueous two-phase system) into which the label of the tally probe is selectively partitioned upon binding of the tally probe to the analyte.

The technology described herein provides a system comprising an integrator probe that is capable of stably associating with an analyte; and a tally probe comprising a label and that is capable of directly or indirectly associating with the analyte, wherein irreversible transfer of the label from the tally probe to the integrator probe occurs more rapidly and/or more efficiently when the tally probe and the integrator probe are both associated with the analyte than when the tally probe and/or the integrator probe is/are dissociated from the analyte. The system may further comprise an adaptor probe that is capable of binding to the analyte and wherein the tally probe indirectly associates with the analyte by directly associating with the adaptor probe. The tally probe may be specific for the analyte and may directly associate with the analyte. The integrator probe may be specific for the analyte. The tally probe may transiently bind the analyte. The tally probe may transiently bind an adaptor probe. The integrator probe may be configured to bind more than one label. The system may comprise an integrator probe comprising multiple labels. The system may further comprise an analyte. The integrator probe may be configured to bind a label non-covalently. The integrator probe may be configured to bind a label covalently. The analyte may comprise a nucleic acid, a protein, a metabolite, a small molecule, a lipid, or a sugar. The integrator probe may comprise a separate phase of matter. The separate phase of matter may be a colloidal particle, liposome, micelle, or emulsified droplet. The integrator probe may comprise a nucleic acid or a protein. The tally probe may comprise a nucleic acid or a protein. The label may be a fluorescent or luminescent label. The system may further comprise a tally probe release component that converts a tally probe bound to the analyte to a dissociated tally probe. The tally probe release component may comprise an enzyme specific for a tally probe-analyte complex. The integrator probe may comprise a first functional group and the label of the tally probe may comprise a second functional group that is reactive with the first functional group. The integrator probe may comprise a first click reactant and the label of the tally probe may comprise a second click reactant. Systems may further comprise a detection component configured to detect and/or quantify a signal produced by an integrator probe comprising multiple labels.

The technology described herein relates to methods for detecting and/or quantifying an analyte. For example, methods may comprise providing a sample comprising an analyte; providing an integrator probe specific for the analyte; and providing a tally probe specific for the analyte and comprising a label. The label may be irreversibly (e.g., substantially or effectively irreversibly) transferred from the tally probe to the integrator probe when the integrator probe and the tally probe are both bound to the analyte. The integrator probe may stably bind the analyte. The tally probe may transiently bind the analyte. The integrator probe may be configured to bind more than one label (e.g., 2, 3, 4, 5, 6, 7, 8, 9, 10, 11, 12, 13, 14, 15, 16, 17, 18, 19, or 20, or more labels). The integrator probe may be configured to bind a label non-covalently (e.g., irreversibly, substantially irreversibly, and/or effectively irreversibly non-covalently). The integrator probe may be configured to bind a label covalently (e.g., irreversibly, substantially irreversibly, and/or effectively irreversibly covalently). The analyte may comprise a nucleic acid, a protein, a metabolite, a small molecule, a lipid, or a sugar. The integrator probe may comprise a nucleic acid or a protein (e.g., an antibody) or both a nucleic acid and a protein. The tally probe may comprise a nucleic acid or a protein (e.g., an antibody) or both a nucleic acid and a protein. The label may be a fluorescent or luminescent label. The methods may further comprise providing a tally probe release component that converts a tally probe bound to the analyte to a dissociated tally probe. The tally probe release component may comprise an enzyme specific for a tally probe-analyte complex. The integrator probe may comprise a first functional group and the label of the tally probe may comprise a second functional group that is selectively reactive towards the first functional group. The integrator probe may comprise a first click reactant and the label of the tally probe may comprise a second click reactant. The integrator probe may comprise a first affinity ligand and the label of the tally probe may comprise a second affinity ligand that selectively binds the first affinity ligand. The integrator probe may comprise a first nucleic acid sequence and the label of the tally probe may comprise a second nucleic acid sequence complementary to the first nucleic acid sequence. The transfer of the label from the tally probe to the integrator probe may occur via toehold-mediated strand displacement. The integrator probe may comprise a consensus target amino acid or nucleic acid sequence, and the label of the tally probe may comprise a ligand that is enzymatically transferred to the consensus target sequence. The integrator probe may comprise a separate solid or liquid phase (e.g., a colloidal particle, droplet of a liquid-in-liquid emulsion, or phase of an aqueous two-phase system) into which the label of the tally probe is selectively partitioned upon binding of the tally probe to the analyte. The analyte may be a biomarker for a disease. The analyte may be a biomarker for a cancer. The sample may be a biofluid. The sample may comprise and/or may be prepared from blood, urine, mucus, saliva, semen, or tissue. Detecting and/or quantifying an analyte in the sample may indicate that the subject has the disease. The methods may further comprise providing a result describing the presence and/or quantity of the analyte in the sample. The methods may further comprise providing a positive control and/or a negative control. The methods may further comprise providing a standard curve.

Additionally disclosed herein is providing a sample comprising an analyte; providing an integrator probe that is capable of stably associating with an analyte; and providing a tally probe comprising a label and that is capable of directly or indirectly associating with the analyte. Irreversible transfer of the label from the tally probe to the integrator probe may occur more rapidly and/or more efficiently when the tally probe and the integrator probe are both associated with the analyte than when the tally probe and/or the integrator probe is/are dissociated from the analyte. The methods may further comprise providing an adaptor probe that is capable of binding to the analyte and wherein the tally probe indirectly associates with the analyte by directly associating with the adaptor probe. The tally probe may be specific for the analyte and may directly associate with the analyte. The integrator probe may be specific for the analyte. The tally probe may transiently bind the analyte. The tally probe may transiently bind the adaptor probe. The integrator probe may be configured to bind more than one label. The integrator probe may be configured to bind a label non-covalently. The integrator probe may be configured to bind a label covalently. The analyte may comprise a nucleic acid, a protein, a metabolite, a small molecule, a lipid, or a sugar. The integrator probe may comprise a separate phase of matter. The separate phase of matter may be a colloidal particle, liposome, micelle, or emulsified droplet. The integrator probe may comprise a nucleic acid or a protein. The tally probe may comprise a nucleic acid or a protein. The label may be a fluorescent or luminescent label. The methods may further comprise providing a tally probe release component that converts a tally probe bound to the analyte to a dissociated tally probe. The tally probe release component may comprise an enzyme specific for a tally probe-analyte complex. The integrator probe may comprise a first functional group and the label of the tally probe may comprise a second functional group that is reactive with the first functional group. The integrator probe may comprise a first click reactant and the label of the tally probe may comprise a second click reactant. The analyte may be a biomarker for a disease and/or a biomarker for a cancer. The sample may be a biofluid. The sample may comprise and/or may be prepared from blood, urine, mucus, saliva, semen, or tissue. Detecting and/or quantifying an analyte in the sample may indicate that the subject has the disease. The methods may further comprise providing a result describing the presence and/or quantity of the analyte in the sample. The methods may further comprise providing a positive control and/or a negative control. The methods may further comprise providing a standard curve.

The technology relates to use of a composition as described herein to detect and/or quantify an analyte in a sample. The technology relates to use of a system as described herein to detect and/or quantify an analyte in a sample.

### BRIEF DESCRIPTION OF THE DRAWINGS

These and other features, aspects, and advantages of the present technology will become better understood with regard to the following drawings:

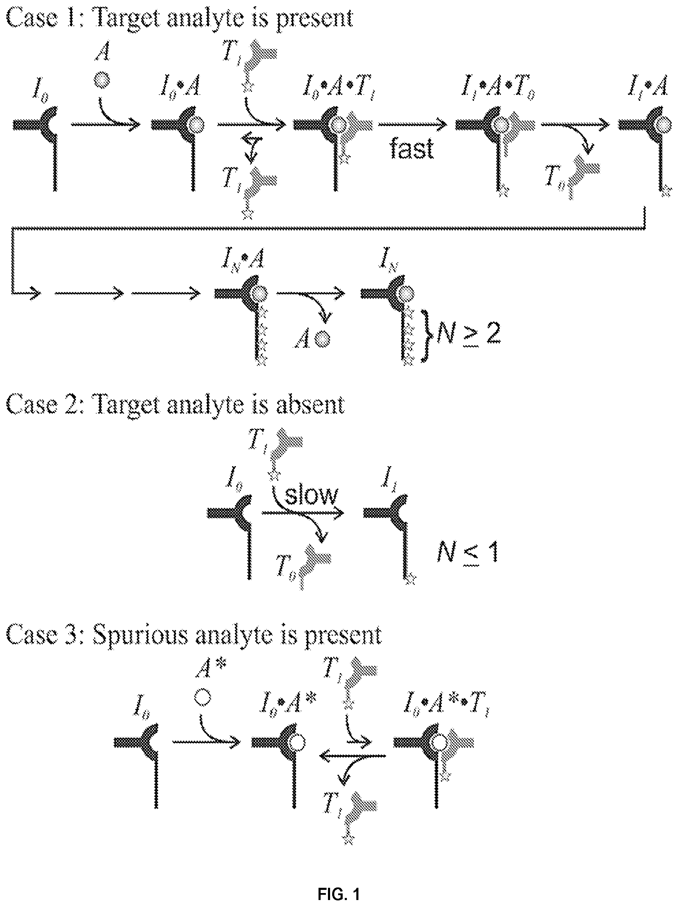

FIG. 1 is a schematic drawing showing the principle of the technology provided herein. An integrator probe (I) binds stably to target analyte (A) and repeated binding of tally probe (T) to the same copy of A triggers the transfer of a label L (yellow star) from T to I. Initially, I comprises no labels and is designated I₀. After multiple cycles of T binding to A, I is modified by N ≥ 2 labels and is designated I_{N}. In the presence of a spurious analyte (A*), binding of T and/or I to A* is weak and transfer of labels from T to I is thus much slower than in the presence of A. The presence of multiple (e.g., greater than 2) labels on a given copy of I provides a specific signature indicating the presence and/or quantity of A in the mixture.

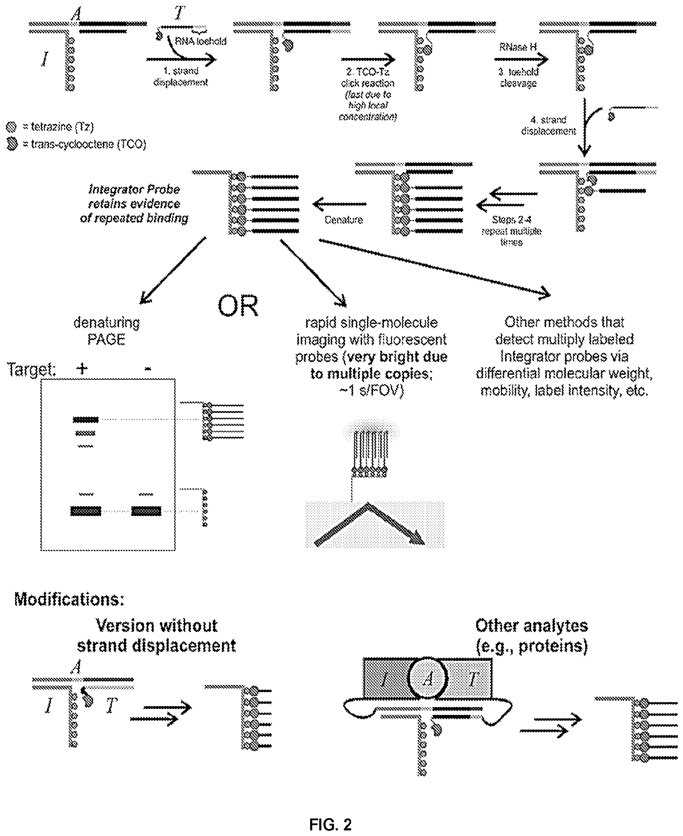

FIG. 2 is a schematic drawing showing the technology using copper-free click chemistry and RNase H for detecting a nucleic acid analyte. Integrator probe I comprises multiple tetrazine (Tz, orange hexagon) moieties and binds stably to A by Watson-Crick base pairing. Tally probe T comprises a single trans-cyclooctene (TCO, green octagon) moiety and binds transiently to A because T comprises a series of RNA nucleotides complementary to A that are selectively degraded by RNase H after binding to A. Due to the high local effective concentration of the TCO and Tz labels when I and T are bound to the same copy of A, a click reaction covalently linking T and I occurs before degradation by RNase H occurs. After RNase H partially degrades the bound copy of T, the number of complementary base pairs between T and A is reduced and a new copy of T hybridizes to A. After multiple cycles of binding, click reaction, and degradation, multiple copies of partially degraded T (comprising the label L) are covalently attached to the associated copy of I, retaining a permanent memory of the occurrence of multiple binding events to the same copy of A. The presence of multiply-labeled integrator probes provides a specific signature indicating the presence and/or quantity of A in the mixture, which can be detected by a detector such as denaturing polyacrylamide electrophoresis (PAGE), fluorescence microscopy, mass spectrometry, and/or chromatography. Other systems may utilize T probes primarily comprising RNA nucleotides (lower left) and/or conjugated to affinity probes that are specific for other types of analytes (such as proteins).

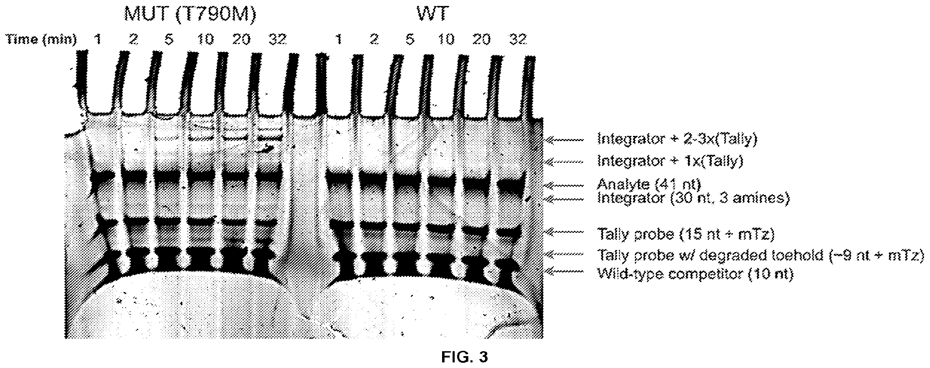

FIG. 3 is a stained denaturing polyacrylamide gel showing results from an experiment in which a point mutation is detected in a 41-nucleotide mutant nucleic acid (MUT) using an integrator probe comprising up to 3 copies of TCO and a tally probe comprising methyltetrazine. The corresponding wild-type (WT) sequence, which lacks the point mutation, is not detected, showing selectivity of the assay for the point mutation.

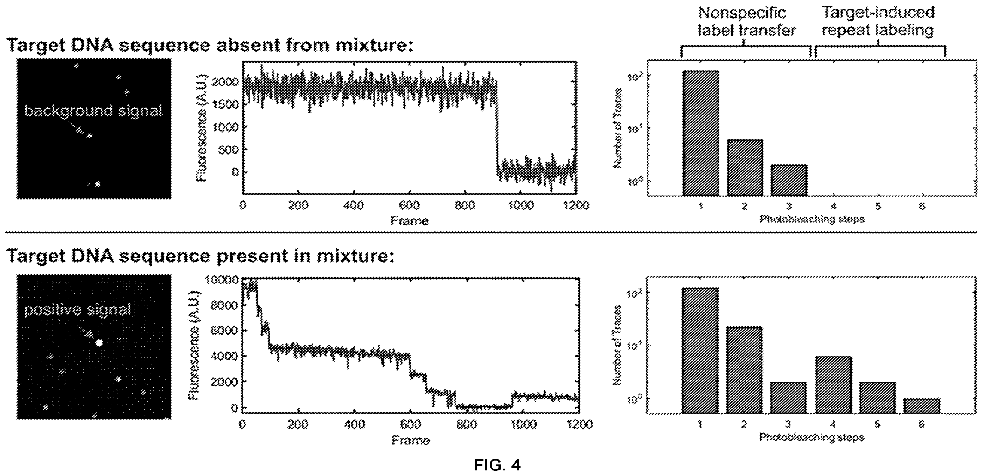

FIG. 4 shows data from an experiment in which the presence of an analyte DNA sequence is detected by single-molecule total internal reflection fluorescence (TIRF) microscopy.

It is to be understood that the figures are not necessarily drawn to scale, nor are the objects in the figures necessarily drawn to scale in relationship to one another. The figures are depictions that are intended to bring clarity and understanding to various apparatuses, systems, and methods disclosed herein. Wherever possible, the same reference numbers will be used throughout the drawings to refer to the same or like parts.

### DETAILED DESCRIPTION

The invention is set out in the appended claims. Provided herein is technology relating to analyte detection and particularly, but not exclusively, to compositions, methods, systems, kits, and uses of the foregoing for detecting, characterizing, identifying, and/or quantifying analytes (e.g., nucleic acids, proteins, or other biomolecules) using molecular probes (e.g., integrator probes) that integrate cumulative repeated binding of probes (e.g., tally probes) to the same analyte molecule. During the development of the technology, experiments were conducted to develop and test a system for detecting a DNA point mutation using labels that were attached to an integrator probe via copper-free click chemistry and visualized by fluorescent staining following gel electrophoresis. Integrator probes comprising multiple labels are detected using single-molecule methods or other detection methods. The label can be any detectable molecular moiety such as a fluorophore, DNA sequence, affinity tag, mass tag, enzyme, hapten, protein tag, etc. and can be attached to the integrator probe covalently or non-covalently. The integrator probe may be, for example, an oligonucleotide, polypeptide, colloidal nanoparticle, or a separate phase within a system comprising multiple liquid phases, with the capability of accepting multiple (e.g., at least two) copies of the label upon repeated binding of the tally probe to the same molecule of analyte.

In this detailed description, for purposes of explanation, numerous specific details are set forth to provide a thorough understanding of the disclosure. One skilled in the art will appreciate, however, that the technology disclosed herein may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied.

Unless defined otherwise, all technical and scientific terms used herein have the same meaning as is commonly understood by one of ordinary skill in the art. When definitions of terms in herein cited references appear to differ from the definitions provided in the present teachings, the definition provided in the present teachings shall control. The section headings used herein are for organizational purposes.

### Definitions

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, as long as they fall within the scope of the claims.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, the terms "about", "approximately", "substantially", and "significantly" are understood by persons of ordinary skill in the art and will vary to some extent on the context in which they are used. For example, the term "substantially" as used herein, is a broad term and is used in its ordinary sense, including, but not limited to, being largely but not necessarily wholly that which is specified. If there are uses of these terms that are not clear to persons of ordinary skill in the art given the context in which they are used, "about" and "approximately" mean plus or minus less than or equal to 10% of the particular term and "substantially" and "significantly" mean plus or minus greater than 10% of the particular term.

As used herein, disclosure of ranges includes disclosure of all values and further divided ranges within the entire range, including endpoints and sub-ranges given for the ranges.

As used herein, the suffix "-free" refers to technology that omits the feature of the base root of the word to which "-free" is appended. That is, the term "X-free" as used herein means "without X", where X is a feature of the technology omitted in the "X-free" technology. For example, a "calcium-free" composition does not comprise calcium, a "mixing-free" method does not comprise a mixing step, etc.

Although the terms "first", "second", "third", etc. may be used herein to describe various steps, elements, compositions, components, regions, layers, and/or sections, these steps, elements, compositions, components, regions, layers, and/or sections should not be limited by these terms, unless otherwise indicated. These terms are used to distinguish one step, element, composition, component, region, layer, and/or section from another step, element, composition, component, region, layer, and/or section. Terms such as "first", "second", and other numerical terms when used herein do not imply a sequence or order unless clearly indicated by the context. Thus, a first step, element, composition, component, region, layer, or section discussed herein could be termed a second step, element, composition, component, region, layer, or section without departing from technology.

As used herein, the word "presence" or "absence" (or, alternatively, "present" or "absent") is used in a relative sense to describe the amount or level of a particular entity (e.g., an analyte). For example, when an analyte is said to be "present" in a test sample, it means the level or amount of this analyte is above a pre-determined threshold; conversely, when an analyte is said to be "absent" in a test sample, it means the level or amount of this analyte is below a pre-determined threshold. The pre-determined threshold may be the threshold for detectability associated with the particular test used to detect the analyte or any other threshold. When an analyte is "detected" in a sample it is "present" in the sample; when an analyte is "not detected" it is "absent" from the sample. Further, a sample in which an analyte is "detected" or in which the analyte is "present" is a sample that is "positive" for the analyte. A sample in which an analyte is "not detected" or in which the analyte is "absent" is a sample that is "negative" for the analyte.

As used herein, the term "detect" refers to detecting the presence or absence of an analyte or the activity or effect of an analyte or for detecting the presence or absence of a variant of an analyte

As used herein, the term "detection assay" refers to an assay for detecting the presence or absence of an analyte or the activity or effect of an analyte or for detecting the presence or absence of a variant of an analyte.

As used herein, the term "analyte" refers to a substance to be tested, assayed, detected, imaged, characterized, described, and/or quantified. Exemplary analytes include, but are not limited to, molecules, atoms, ions, biomolecules (e.g., nucleic acids (e.g., DNA, RNA) as described and as defined herein), polypeptides (e.g., peptides, proteins, glycoproteins), carbohydrates, lipids, amino acids, small molecules (drugs, bioactive agents, toxins, cofactors, metabolites), etc.

As used herein, an "increase" or a "decrease" refers to a detectable (e.g., measured) positive or negative change, respectively, in the value of a variable relative to a previously measured value of the variable, relative to a pre-established value, and/or relative to a value of a standard control. An increase is a positive change preferably at least 10%, more preferably 50%, still more preferably 2-fold, even more preferably at least 5-fold, and most preferably at least 10-fold relative to the previously measured value of the variable, the pre-established value, and/or the value of a standard control. Similarly, a decrease is a negative change preferably at least 10%, more preferably 50%, still more preferably at least 80%, and most preferably at least 90% of the previously measured value of the variable, the pre-established value, and/or the value of a standard control. Other terms indicating quantitative changes or differences, such as "more" or "less," are used herein in the same fashion as described above.

As used herein, a "system" refers to a plurality of real and/or abstract components operating together for a common purpose. A "system" may be an integrated assemblage of hardware and/or software components. Each component of the system may interact with one or more other components and/or may be related to one or more other components. A system may refer to a combination of components and software for controlling and directing methods.

As used herein, the term "sample" is used in its broadest sense. For example, a sample is or comprise an animal cell or tissue. A sample may include a specimen or a culture (e.g., a microbiological culture) obtained from any source, as well as biological and environmental samples. Biological samples may be obtained from plants or animals (including humans) and encompass fluids, solids, tissues, and gases. Environmental samples include, e.g., environmental material such as surface matter, soil, water, and industrial samples, as well as samples obtained from food and dairy processing instruments, apparatuses, equipment, utensils, disposable items, and non-disposable items. These examples are not to be construed as limiting the sample types applicable to the present technology.

As used herein, a "biological sample" refers to a sample of biological tissue or fluid. For instance, a biological sample may be a sample obtained from an animal (including a human); a fluid, solid, or tissue sample; as well as liquid and solid food and feed products and ingredients such as dairy items, vegetables, meat and meat byproducts, and waste. Biological samples may be obtained from any of the various families of domestic animals, as well as feral or wild animals, including, but not limited to, such animals as ungulates, bear, fish, lagomorphs, rodents, etc. Examples of biological samples include sections of tissues, blood, blood fractions, plasma, serum, urine, or samples from other peripheral sources or cell cultures, cell colonies, single cells, or a collection of single cells. Furthermore, a biological sample includes pools or mixtures of the abovementioned samples. A biological sample may be provided by removing a sample of cells from a subject but can also be provided by using a previously isolated sample. For example, a tissue sample can be removed from a subject suspected of having a disease by conventional biopsy techniques. A blood sample may be taken from a subject. A biological sample from a patient means a sample from a subject suspected to be affected by a disease.

As used herein, the term "biofluid" refers to a biological fluid (e.g., a body fluid, a bodily fluid). For example, a biofluids is an excretion (e.g., urine, sweat, exudate (e.g., including plant exudate)) or a biofluid is a secretion (e.g., breast milk, bile). A biofluid may be obtained using a needle (e.g., blood, cerebrospinal fluid, lymph). A biofluid may be produced as a result of a pathological process (e.g., a blister, cyst fluid). A biofluid may be derived from another biofluid (e.g., plasma, serum). Exemplary biofluids include, but are not limited to, amniotic fluid, aqueous humor, vitreous humor, bile, blood, blood plasma, blood serum, breast milk, cerebrospinal fluid, cerumen (earwax), chyle, chime, endolymph, perilymph, exudates, feces, female ejaculate, gastric acid, gastric juice, lymph, mucus (e.g., nasal drainage, phlegm), pericardial fluid, peritoneal fluid, pleural fluid, pus, rheum, saliva, sebum (e.g., skin oil), serous fluid, semen, smegma, sputum, synovial fluid, sweat, tears, urine, vaginal secretion, and vomit.

The term "label" as used herein refers to any atom, molecule, molecular complex (e.g., metal chelate), or colloidal particle (e.g., quantum dot, nanoparticle, microparticle, etc.) that can be used to provide a detectable (e.g., quantifiable) effect, and that can be attached to an integrator probe and transferred to a tally probe (e.g., with or without a portion of the tally probe and/or a linker or portion thereof). Labels are compounds, structures, or elements that are amenable to at least one method of detection and/or isolation that allows for discrimination between different labels. Exemplary labels that find use with the technology provided herein include, for example, a dye, a fluorescent label, a chemiluminescent label, a quencher, a radioactive label, or combinations thereof. Labels include luminogenic and/or luminescent molecules, colored molecules (e.g., chromogens), and scintillants. Labels also include any useful linker molecule (such as biotin, avidin, digoxigenin, streptavidin, HRP, protein A, protein G, antibodies or fragments thereof, Grb2, polyhistidine, Ni²⁺, FLAG tags, myc tags), heavy metals (e.g., gold), enzymes (e.g., alkaline phosphatase, peroxidase, and luciferase), electron donors/acceptors, acridinium esters, and calorimetric substrates. It is also envisioned that a change in mass may be considered a detectable label, e.g., as finds use in surface plasmon resonance detection or mass spectrometry. Labels may provide signals detectable by fluorescence, luminescence, radioactivity, colorimetry, gravimetry, X-ray diffraction or absorption, magnetism, enzymatic activity, characteristics of mass or behavior affected by mass (e.g., mass spectrometry; fluorescence polarization), and the like. A label may be a charged moiety (positive or negative charge) or, alternatively, may be charge neutral. Labels can include or consist of nucleic acid or protein sequence, so long as the sequence comprising the label is detectable. The label may be a fluorescent label (e.g., a fluorophore).

As used herein the term "fluorophore" will be understood to refer to both fluorophores and luminophores and chemical agents that quench fluorescent or luminescent emissions. Further, as used herein, a "fluorophore" refers to any species possessing a fluorescent property when appropriately stimulated. The stimulation that elicits fluorescence is typically illumination; however, other types of stimulation (e.g., collisional) are also considered herein. The terms "fluorophore", "fluor", "fluorescent moiety", "fluorescent dye", and "fluorescent group" are used interchangeably. A fluorescent label may comprise a fluorophore as described below in the section entitled "Fluorescent labels".

As used herein, a "nucleic acid" or a "nucleic acid sequence" refers to a polymer or oligomer of pyrimidine and/or purine bases, preferably cytosine, thymine, and uracil, and adenine and guanine, respectively (See Albert L. Lehninger, Principles of Biochemistry, at 793-800 (Worth Pub. 1982)). The present technology contemplates any deoxyribonucleotide, ribonucleotide, or peptide nucleic acid component, and any chemical variants thereof, such as methylated, hydroxymethylated, or glycosylated forms of these bases, and the like. The polymers or oligomers may be heterogenous or homogenous in composition and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. A nucleic acid or nucleic acid sequence may comprise other kinds of nucleic acid structures such as, for instance, a DNA/RNA helix, peptide nucleic acid (PNA), morpholino, locked nucleic acid (LNA), and/or a ribozyme. Hence, the term "nucleic acid" or "nucleic acid sequence" may also encompass a chain comprising non-natural nucleotides, modified nucleotides, and/or non- nucleotide building blocks that can exhibit the same function as natural nucleotides (e.g., "nucleotide analogs"); further, the term "nucleic acid sequence" as used herein refers to an oligonucleotide, nucleotide or polynucleotide, and fragments or portions thereof, and to DNA or RNA of genomic or synthetic origin, which may be single or double-stranded, and represent the sense or antisense strand.

The term "nucleotide analog" as used herein refers to modified or non-naturally occurring nucleotides including but not limited to analogs that have altered stacking interactions such as 7-deaza purines (e.g., 7-deaza-dATP and 7-deaza-dGTP); base analogs with alternative hydrogen bonding configurations (e.g., such as Iso-C and Iso-G and other non-standard base pairs, e.g., as described in U.S. Pat. No. 6,001,983); non-hydrogen bonding analogs (e.g., non-polar, aromatic nucleoside analogs such as 2,4-difluorotoluene, e.g., as described by Schweitzer and Kool, J. Org. Chem., 1994, 59, 7238-7242, Schweitzer and Kool, J. Am. Chem. Soc., 1995, 117, 1863-1872); "universal" bases such as 5-nitroindole and 3-nitropyrrole; and universal purines and pyrimidines (such as "K" and "P" nucleotides, respectively; P. Kong, et al., Nucleic Acids Res., 1989, 17, 10373-10383, P. Kong et al., Nucleic Acids Res., 1992, 20, 5149-5152). Nucleotide analogs include nucleotides having modification on the sugar moiety, such as dideoxy nucleotides and 2'-O-methyl nucleotides. Nucleotide analogs include modified forms of deoxyribonucleotides as well as ribonucleotides.

"Peptide nucleic acid" means a DNA mimic that incorporates a peptide-like polyamide backbone.

As used herein, the terms "complementary" or "complementarity" are used in reference to polynucleotides (e.g., a sequence of nucleotides such as an oligonucleotide tally probe, oligonucleotide integrator probe, or a target analyte that is a nucleic acid) related by the base-pairing rules. For example, for the sequence "5'-A-G-T-3'" is complementary to the sequence "3'-T-C-A-5'." Complementarity may be "partial", in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions and in detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

In some contexts, the term "complementarity" and related terms (e.g., "complementary" and "complement") refers to the nucleotides of a nucleic acid sequence that can bind to another nucleic acid sequence through hydrogen bonds, e.g., nucleotides that are capable of base pairing, e.g., by Watson-Crick base pairing or other base pairing. Nucleotides that can form base pairs, e.g., that are complementary to one another, are the pairs: cytosine and guanine, thymine and adenine, adenine and uracil, and guanine and uracil. The percentage complementarity need not be calculated over the entire length of a nucleic acid sequence. The percentage of complementarity may be limited to a specific region of which the nucleic acid sequences that are base-paired, e.g., starting from a first base-paired nucleotide and ending at a last base-paired nucleotide. The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids used in the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide(s), base composition of the oligonucleotide(s), sequence of the oligonucleotide(s), ionic strength of a composition comprising the oligonucleotide(s), and incidence of mismatched base pairs between two oligonucleotides.

Thus, "complementary" may refer to a first nucleic acid sequence that is at least 60%, 65%, 70%, 75%, 80%, 85%, 90%, 95%, 97%, 98%, or 99% identical to the complement of a second nucleic acid sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases, or that the two sequences hybridize under stringent hybridization conditions. "Fully complementary" means each nucleobase of a first nucleic acid is capable of pairing with each nucleobase at a corresponding position in a second nucleic acid. For example, an oligonucleotide wherein each nucleobase has complementarity to a nucleic acid has a nucleobase sequence that is identical to the complement of the nucleic acid over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases.

As used herein, the term "mismatch" refers to a nucleobase of a first nucleic acid that is not capable of pairing with a nucleobase at a corresponding position of a second nucleic acid.

As used herein, the term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified", "mutant", "variant", or "polymorphic" refers to a gene or gene product that displays modifications in sequence and/or functional properties (e.g., altered characteristics) when compared to the wild-type gene or gene product. It is noted that naturally-occurring mutants can be isolated; these are identified by the fact that they have altered characteristics when compared to the wild-type gene or gene product. Thus, the terms "variant" and "mutant" when used in reference to a nucleotide sequence refer to a nucleic acid sequence that differs by one or more nucleotides from another, usually related nucleotide acid sequence. A "variation" is a difference between two different nucleotide sequences; one sequence may be a "reference sequence".

As used herein, the term "hybridization" is used in reference to the pairing of complementary nucleic acids. Hybridization and the strength of hybridization (e.g., the strength of the association between the nucleic acids) is influenced by such factors as the degree of complementary between the nucleic acids, stringency of the conditions involved, and the Tₘ of the formed hybrid. "Hybridization" methods involve the annealing of one nucleic acid to another, complementary nucleic acid, e.g., a nucleic acid having a complementary nucleotide sequence. The ability of two polymers of nucleic acid comprising complementary sequences to find each other and anneal through base pairing interaction is a well-recognized phenomenon. The initial observations of the "hybridization" process by Marmur and Lane, Proc. Natl. Acad. Sci. USA 46: 453 (1960) and Doty et al., Proc. Natl. Acad. Sci. USA 46:461 (1960) have been followed by the refinement of this process into an essential tool of modern biology.

As used herein, the term "toehold" refers to a short (e.g., comprising 1-10 (e.g., 1, 2, 3, 4, 5, 6, 7, 8, 9, or 10 nt)) single-stranded nucleic acid extension that is adjacent to a nucleic acid duplex and that accelerates the binding of a third nucleic acid to one of the strands of the duplex and that displaces one strand of the original duplex. Thus, a "toehold" may provide a nucleation site of a nucleic acid that is configured to initiate hybridization of a complementary nucleic acid sequence. The rate constants of hybridization between complementary oligonucleotide sequences can be manipulated over a range of approximately 1 million-fold using a phenomenon known as toehold-mediated strand displacement (see, e.g., Zhang & Winfree (2009) "Control of DNA strand displacement kinetics using toehold exchange." J. Am. Chem. Soc. 131: 17303-14).

As used herein, the term "displacement" encompasses both complete displacement and at least partial displacement. As will be appreciated by one of skill in the art, partial displacement may be sufficient for various teachings herein, and/or could occur before complete displacement occurs. Complete or partial displacement will be adequate for the function to be achieved. Complete or partial displacement can each be specified as desired by the term "complete" or "partial".

As used herein, the term "Tₘ" is used in reference to the "melting temperature." The melting temperature is the temperature at which a population of double-stranded nucleic acid molecules becomes half dissociated into single strands. Several equations for calculating the Tₘ of nucleic acids are well known in the art. As indicated by standard references, a simple estimate of the Tₘ value may be calculated by the equation: Tₘ = 81.5 + 0.41 * (% G+C), when a nucleic acid is in aqueous solution at 1 M NaCl (see, e.g., Anderson and Young, "Quantitative Filter Hybridization" in Nucleic Acid Hybridization (1985). Other references (e.g., Allawi and SantaLucia, Biochemistry 36: 10581-94 (1997)) include more sophisticated computations which account for structural, environmental, and sequence characteristics to calculate Tₘ. For example, these computations may provide an improved estimate of Tₘ for short nucleic acid probes and targets (e.g., as used in the examples).

The terms "protein" and "polypeptide" refer to compounds comprising amino acids joined via peptide bonds and are used interchangeably. A "protein" or "polypeptide" encoded by a gene is not limited to the amino acid sequence encoded by the gene but includes post-translational modifications of the protein. Where the term "amino acid sequence" is recited herein to refer to an amino acid sequence of a protein molecule, "amino acid sequence" and like terms such as "polypeptide" or "protein" are not meant to limit the amino acid sequence to the complete, native amino acid sequence associated with the recited protein molecule. Furthermore, an "amino acid sequence" can be deduced from the nucleic acid sequence encoding the protein. Conventional one-letter and three-letter amino acid codes are used herein as follows - Alanine: Ala, A; Arginine: Arg, R; Asparagine: Asn, N; Aspartate: Asp, D; Cysteine: Cys, C; Glutamate: Glu, E; Glutamine: Gln, Q; Glycine: Gly, G; Histidine: His, H; Isoleucine: Ile, I; Leucine: Leu, L; Lysine: Lys, K; Methionine: Met, M; Phenylalanine: Phe, F; Proline: Pro, P; Serine: Ser, S; Threonine: Thr, T; Tryptophan: Trp, W; Tyrosine: Tyr, Y; Valine: Val, V. As used herein, the codes Xaa and X refer to any amino acid.

The terms "variant" and "mutant" when used in reference to a polypeptide refer to an amino acid sequence that differs by one or more amino acids from another, usually related polypeptide.

As used herein, a "stable interaction" or referring to a "stably bound" interaction refers to an association of two entities or components that is relatively persistent under the thermodynamic equilibrium conditions of the interaction. A "stable interaction" may be an interaction between two components having a K_{D} that is smaller than approximately 10⁻⁹ M or a K_{D} that may be smaller than 10⁻⁸ M. A "stable interaction" may have a dissociation rate constant k_{off} that is smaller than 1 per hour or a dissociation rate constant k_{off} that may be smaller than 1 per minute. A "stable interaction" may be defined as not being a "transient interaction" and a "transient interaction" is defined as not being a "stable interaction". A "stable interaction" may include interactions mediated by covalent bonds and other interactions that are not typically described by a K_{D} value but that involve an average association lifetime between two entities that is longer than approximately 1 minute (e.g., 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, or 180 seconds) per each interaction.

The distinction between a "stable interaction" and a "transient interaction" may be determined by a cutoff value of K_{D} and/or k_{off} and/or another kinetic or thermodynamic value describing the associations, wherein the cutoff is used to discriminate between stable and transient interactions that might otherwise be characterized differently if described in absolute terms of a K_{D} and/or k_{off} or another kinetic or thermodynamic value describing the associations. For example, a "stable interaction" characterized by a K_{D} value might also be characterized as a "transient interaction" in the context of another interaction that is even more stable. One of skill in the art would understand other relative comparisons of stable and transient interactions, e.g., that a "transient interaction" characterized by a K_{D} value might also be characterized as a "stable interaction" in the context of another interaction that is even more transient (less stable).

As used herein, the terms "stable interaction", "stable binding", and "stable association" are used interchangeably. As used herein, the terms "transient interaction", "transient binding", and "transient association" are used interchangeably.

The terms "specific binding" or "specifically binding" when used in reference to the interaction of two components A and B that associate with one another refers to an association of A and B having a K_{D} that is smaller than the K_{D} for the interaction of A or B with other similar components in the solution, e.g., at least one other molecular species in the solution that is not A or B.

As used herein, the term "affinity" refers to the strength of interaction (e.g., binding) of one entity (e.g., molecule) with another entity (e.g., molecule), e.g., an antibody with an antigen. Affinity may depend on the closeness of stereochemical fit between entities, on the size of the area of contact between them, on the distribution of charged and hydrophobic groups, etc.

As used herein, the term "irreversible interaction" refers to an interaction (e.g., association, binding, etc.) having a dissociation half-life longer than the incubation time, e.g., a time that is 1 to 10 minutes (e.g., 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, or 600 seconds, or longer).

As used herein, the term "sensitivity" refers to the probability that an assay gives a positive result for the analyte when the sample comprises the analyte. Sensitivity is calculated as the number of true positive results divided by the sum of the true positives and false negatives. Sensitivity is a measure of how well an assay detects an analyte.

As used herein, the term "specificity" refers to the probability that an assay gives a negative result when the sample does not comprise the analyte. Specificity is calculated as the number of true negative results divided by the sum of the true negatives and false positives. Specificity is a measure of how well a method of the present invention excludes samples that do not comprise an analyte from those that do comprise the analyte.

As used herein, the "equilibrium constant" (K_{eq}), the "equilibrium association constant" (Kₐ), and "association binding constant" (or "binding constant" (K_{B})) are used interchangeably for the following binding reaction of A and B at equilibrium: 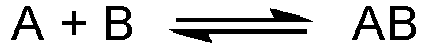 where A and B are two entities that associate with each other (e.g., tally probe and analyte, integrator probe and analyte, label and integrator probe, label and tally probe, etc.) and K_{eq} = [AB] / ([A] × [B]). The dissociation constant K_{D} = 1/K_{B}. The K_{D} is a useful way to describe the affinity of a one binding partner A for a partner B with which it associates, e.g., the number K_{D} represents the concentration of A or B that is required to yield a significant amount of AB. K_{eq} = k_{off} / kₒₙ; K_{D} = k_{off} / kₒₙ. Accordingly, the dissociation constant, K_{D}, and the association constant, K_{A}, are quantitative measures of affinity. At equilibrium, A and B are in equilibrium with A-B complex, and the rate constants, kₐ and k_{d}, quantify the rates of the individual forward and backward reactions of the equilibrium state: 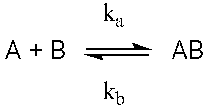 At equilibrium, kₐ [A][B] = k_{d} [AB]. The dissociation constant, K_{D}, is given by K_{D} = k_{d} / kₐ = [A][B] / [AB]. K_{D} has units of concentration, e.g., M, mM, µM, nM, pM, etc. When comparing affinities expressed as K_{D}, a greater affinity is indicated by a lower value. The association constant, K_{A}, is given by K_{A} = K_{A} / K_{D} = [AB] / [A][B]. K_{A} has units of inverse concentration, most typically M⁻¹, mM⁻¹, µM⁻¹, nM⁻¹, pM⁻¹, etc.

As used herein, "moiety" refers to one of two or more parts into which something may be divided, such as, for example, the various parts of an oligonucleotide, a molecule, a chemical group, a domain, a probe, an "R" group, a polypeptide, etc.

The technology may comprise an antibody component or moiety, e.g., an antibody or fragments or derivatives thereof. As used herein, an "antibody", also known as an "immunoglobulin" (e.g., IgG, IgM, IgA, IgD, IgE), comprises two heavy chains linked to each other by disulfide bonds and two light chains, each of which is linked to a heavy chain by a disulfide bond. The specificity of an antibody resides in the structural complementarity between the antigen combining site of the antibody (or paratope) and the antigen determinant (or epitope). Antigen combining sites are made up of residues that are primarily from the hypervariable or complementarity determining regions (CDRs). Occasionally, residues from non-hypervariable or framework regions influence the overall domain structure and hence the combining site. A fragment of an antibody may be comprised, e.g., any protein or polypeptide-containing molecule that comprises at least a portion of an immunoglobulin molecule such as to permit specific interaction between said molecule and an antigen. The portion of an immunoglobulin molecule may include, but is not limited to, at least one complementarity determining region (CDR) of a heavy or light chain or a ligand binding portion thereof, a heavy chain or light chain variable region, a heavy chain or light chain constant region, a framework region, or any portion thereof. Such fragments may be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. The various portions of antibodies can be joined together chemically by conventional techniques or can be prepared as a contiguous protein using genetic engineering techniques.

Fragments of antibodies include, but are not limited to, Fab (e.g., by papain digestion), F(ab')₂ (e.g., by pepsin digestion), Fab' (e.g., by pepsin digestion and partial reduction) and Fv or scFv (e.g., by molecular biology techniques) fragments. A Fab fragment can be obtained by treating an antibody with the protease papaine. Also, the Fab may be produced by inserting DNA encoding a Fab of the antibody into a vector for prokaryotic expression system or for eukaryotic expression system and introducing the vector into a prokaryote or eukaryote to express the Fab. A F(ab')₂ may be obtained by treating an antibody with the protease pepsin. Also, the F(ab')₂ can be produced by binding a Fab' via a thioether bond or a disulfide bond. A Fab may be obtained by treating F(ab')₂ with a reducing agent, e.g., dithiothreitol. Also, a Fab' can be produced by inserting DNA encoding a Fab' fragment of the antibody into an expression vector for a prokaryote or an expression vector for a eukaryote and introducing the vector into a prokaryote or eukaryote for its expression. A Fv fragment may be produced by restricted cleavage by pepsin, e.g., at 4°C and pH 4.0. (a method called "cold pepsin digestion"). The Fv fragment consists of the heavy chain variable domain (V_{H}) and the light chain variable domain (V_{L}) held together by strong noncovalent interaction. A scFv fragment may be produced by obtaining cDNA encoding the V_{H} and V_{L} domains as previously described, constructing DNA encoding scFv, inserting the DNA into an expression vector for prokaryote or an expression vector for eukaryote, and then introducing the expression vector into a prokaryote or eukaryote to express the scFv.

In general, antibodies can usually be raised to any antigen, using the many conventional techniques now well known in the art.

As used herein, the term "conjugated" refers to when one molecule or agent is physically or chemically coupled or adhered to another molecule or agent. Examples of conjugation include covalent linkage and electrostatic complexation. The terms "complexed," "complexed with," and "conjugated" are used interchangeably herein.

As used herein, the term "incubation temperature" refers to a temperature at which a composition comprising an integrator probe, a tally probe, and a sample is incubated (e.g., held or maintained constant or substantially constant or effectively constant) prior to observing the composition to detect, characterize, and/or quantify the analyte, if present, in the sample (e.g., to record data indicating the presence, absence, and/or quantity of analyte, if present, in the sample). The "incubation temperature" may be controlled using active heating and cooling (e.g., a heat pump, Peltier device, water bath, or other technology known in the art to maintain the temperature of a sample, e.g., within 0.1 to 0.5 to 1.0°C of a set temperature). The incubation temperature may be 10 to 110°C (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, or 110°C).

As used herein, the term "incubation time" refers to a length of time for which a composition comprising an integrator probe, a tally probe, and a sample is incubated (e.g., held or maintained constant or substantially constant or effectively constant) at the incubation temperature prior to observing the composition to detect, characterize, and/or quantify the analyte, if present, in the sample (e.g., to record data indicating the presence, absence, and/or quantity of analyte, if present, in the sample). The incubation time is sufficient for multiple cycles of a tally probe binding to an analyte molecule and label transfer from a tally probe to an integrator probe to produce one or more copies of the integrator probe that is/are labeled with multiple copies of the label. The "incubation time" may refer to an amount of time that is tens to hundreds to thousands of seconds, e.g., 5, 10, 15, 20, 25, 30, 35, 40, 45, 50, 55, or 60 seconds; e.g., 5, 10, 15, 20, 25, 30, 35, 40, 45, 50, 55, or 60 minutes; e.g., 1, 1.5, 2, 2.5, or 3 hours. The "incubation time" may refer to an amount of time that is approximately 1 to 10 seconds to 1 to 10 minutes (e.g., approximately 1 to 100 seconds, e.g., 1, 2, 3, 4, 5, 6, 7, 8, 9, 10, 11, 12, 13, 14, 15, 16, 17, 18, 19, 20, 30, 40, 50, 60, 70, 80, 90, or 100 seconds, e.g., 1 to 100 minutes, e.g., 1, 2, 3, 4, 5, 6, 7, 8, 9, 10, 11, 12, 13, 14, 15, 16, 17, 18, 19, 20, 30, 40, 50, 60, 70, 80, 90, or 100 minutes. The incubation time may comprise multiple shorter incubation times (e.g., a first incubation time, a second incubation time, a third incubation time, etc.) at different incubation temperatures (e.g., a first incubation temperature, a second incubation temperature, a third incubation temperature, etc.)

As used herein, the term "integrator probe" refers to any entity (e.g., molecule, biomolecule, colloidal particle, liquid phase, etc.) that binds stably to an analyte and that can irreversibly (e.g., substantially irreversibly and/or effectively irreversibly) accept (e.g., stably bind covalently or stably bind non-covalently) multiple labels from one or more tally probes. The integrator probe may bind an analyte with high thermodynamic stability (e.g., with a melting temperature more than 10 degrees C above the incubation temperature or an average lifetime longer than the incubation time). The integrator probe may be covalently linked to a plurality of labels. The integrator probe may be indirectly and/or non-covalently linked and/or associated with a plurality of labels. The integrator probe may be a protein that recognizes an analyte. The integrator probe may be a nucleic acid (e.g., an oligonucleotide) that recognizes an analyte. For example, the integrator probe comprises, e.g., DNA, RNA, a nucleic acid comprising DNA and RNA, a nucleic acid comprising modified bases and/or modified linkages between bases such as, e.g., a nucleic acid as described herein. The nucleic acid integrator probe may comprise a nucleic acid aptamer. The integrator probe may comprise more than one type of molecule (e.g., more than one of a protein, a nucleic acid, a chemical linker, or a chemical moiety). For example, the integrator probe comprises a conjugate comprising a protein and a nucleic acid (e.g., a protein-nucleic acid conjugate in which a nucleic acid (e.g., an oligonucleotide) is covalently linked to a polypeptide (e.g., a protein and/or a peptide) to provide a chimeric molecule. The integrator probe may comprise an antibody (e.g., a monoclonal antibody) or antibody fragment. The integrator probe may comprise a nanobody, a DNA-binding protein, or a DNA-binding protein domain. The integrator probe may be a small organic molecule (e.g., a molecule having a molecular weight that is less than approximately 2000 daltons, e.g., less than 2100, 2050, 2000, 1950, 1900, 1850, 1800, 1750, 1700, 1650, 1600, 1550, 1500 daltons, or less). The integrator probe may be labeled prior to associating with an analyte (e.g., the integrator probe may be labeled with a first label and can irreversibly accept (e.g., stably bind covalently or non-covalently) multiple second labels from one or more tally probes). If the analyte comprises a carbohydrate or polysaccharide, the integrator probe may comprise a carbohydrate-binding protein such as a lectin or a carbohydrate-binding antibody. The integrator probe may be engineered to strengthen its binding relative to the non-engineered version, e.g., to provide a binding and/or association with an analyte that is more thermodynamically stable than the non-engineered version. In addition to integrator probes comprising antibodies, target analytes and integrator probes that are proteins may be comprised, e.g., where one binding partner is the integrator probe and the other binding partner is the analyte being measured. The use of aptamers binding any ligand, lectins binding glycosylated proteins, proteins or other molecules binding lipids, etc. may be comprised. The technology comprises the use of any binding pair with stable binding behavior suitable for detection as described herein.

As used herein, the term "tally probe" refers to any entity (e.g., molecule, biomolecule, etc.) that binds transiently to an analyte and that comprises a label that can be irreversibly (e.g., substantially irreversibly or effectively irreversibly) transferred from the tally probe to an integrator probe. The tally probe may have a dissociation constant (K_{D}) for the analyte of larger than approximately 0.1 nanomolar (e.g., greater than 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 or more nanomolar) under the assay conditions (e.g., incubation temperature, probe concentrations, pH, salt concentration, and/or the presence of a tally probe release component). The technology contemplates a broad range of analyte types, tally probe types, and associated types and/or strengths of transient interactions between the tally probe and the analyte. Accordingly, the tally probe may have a dissociation constant (K_{D}) for the analyte that is in the range of approximately 0.1 nm to approximately 100 nm (e.g., 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 6.0, 7.0, 8.0, 9.0, 10, 15, 20, 25, 30, 35, 40, 45, 50, 55, 60, 65, 70, 75, 80, 85, 90, 95, or 100 nm) under the assay conditions (e.g., incubation temperature, probe concentrations, pH, salt concentration, and/or the presence of a tally probe release component). If a tally probe has more than one form (e.g., is converted from a first form (e.g., a form bound to an analyte) to another form (e.g., a form that dissociates from the analyte)), e.g., by a tally probe release component, the first form of the tally probe may have a first dissociation constant (K_{D}) for the analyte that is in the range of approximately 1 to approximately 100 picomolar (e.g., 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 6.0, 7.0, 8.0, 9.0, 10, 15, 20, 25, 30, 35, 40, 45, 50, 55, 60, 65, 70, 75, 80, 85, 90, 95, or 100 nm) under the assay conditions (e.g., incubation temperature, probe concentrations, pH, salt concentration, and/or the presence of a tally probe release component) and the second form of the tally probe may have a second dissociation constant (K_{D}) for the analyte that is in the range of approximately 10 nm to approximately 1000 nm (e.g., 10, 20, 30, 40, 50, 60, 70, 80, 90, 100, 200, 300, 400, 500, 600, 700, 800, 900, or 1000 nm) under the assay conditions (e.g., incubation temperature, probe concentrations, pH, salt concentration, and/or the presence of a tally probe release component).

The tally probe may have a binding rate constant and/or a dissociation rate constant for the analyte that is larger than approximately 0.1 min⁻¹ (e.g., greater than 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 or more min⁻¹) under the assay conditions (e.g., incubation temperature, probe concentrations, pH, salt concentration, and/or the presence of a tally probe release component). The technology contemplates a broad range of analyte types, tally probe types, and associated types and/or strengths of transient interactions between the tally probe and the analyte. Accordingly, the tally probe may have a binding rate constant and/or a dissociation rate constant for the analyte that is in the range of approximately 0.1 min⁻¹ to 10 min⁻¹ (e.g., 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0 min⁻¹) under the assay conditions (e.g., incubation temperature, probe concentrations, pH, salt concentration, and/or the presence of a tally probe release component). If a tally probe has more than one form (e.g., is converted from a first form (e.g., a form bound to an analyte) to another form (e.g., a form that dissociates from the analyte)), e.g., by a tally probe release component, the first form of the tally probe may have a first dissociation rate constant for the analyte that is approximately less than 0.1 min⁻¹ (e.g., less than 0.10, 0.09, 0.08, 0.07, 0.06, 0.05, 0.04, 0.03, 0.02, 0.01, 0.010, 0.009, 0.008, 0.007, 0.006, 0.005, 0.004, 0.003, 0.002, or 0.001 min⁻¹) under the assay conditions (e.g., incubation temperature, probe concentrations, pH, salt concentration, and/or the presence of a tally probe release component) and the second form of the tally probe may have a second dissociation rate constant for the analyte that is in the range of approximately 0.1 min⁻¹ to approximately 10 min⁻¹ (e.g., 10.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0 min⁻¹) under the assay conditions (e.g., incubation temperature, probe concentrations, pH, salt concentration, and/or the presence of a tally probe release component). The binding rate constant of the tally probe with the analyte may be at least 10⁵ to 10⁶ M⁻¹ s⁻¹.

The transient binding of a tally probe may result from converting a stably bound tally probe into a transiently bound tally probe that dissociates from the analyte, e.g., by an enzymatic reaction or change in conformation that converts a tally probe stably bound to an analyte into a transiently bound tally probe that dissociates from the analyte. The tally probe may be covalently linked to a label that can be transferred to an integrator probe. The tally probe may be indirectly and/or non-covalently linked and/or associated with a label that can be transferred to an integrator probe. The tally probe may be a protein that recognizes an analyte. The tally probe may be a nucleic acid (e.g., an oligonucleotide) that recognizes an analyte. For example, the tally probe comprises, e.g., DNA, RNA, a nucleic acid comprising DNA and RNA, a nucleic acid comprising modified bases and/or modified linkages between bases such as, e.g., a nucleic acid as described herein. A tally probe may comprise a first portion comprising DNA nucleotides and a second portion comprising RNA nucleotides. A tally probe may comprise DNA nucleotides and RNA nucleotides in a ratio of approximately 10:1, 5:1, 2:1, 1:1, 1:2, 1:5, or 1:10. The nucleic acid tally probe may comprise a nucleic acid aptamer. The tally probe may comprise more than one type of molecule (e.g., more than one of a protein, a nucleic acid, a chemical linker, or a chemical moiety). The tally probe may comprise an antibody (e.g., a monoclonal antibody) or antibody fragment. The tally probe may comprise a low-affinity antibody (e.g., monoclonal antibody) or antibody fragment. The tally probe may comprise a nanobody, a DNA-binding protein, or a DNA-binding protein domain. The tally probe may be a small organic molecule (e.g., a molecule having a molecular weight that is less than approximately 2000 daltons, e.g., less than 2100, 2050, 2000, 1950, 1900, 1850, 1800, 1750, 1700, 1650, 1600, 1550, 1500 daltons, or less). If the analyte comprises a carbohydrate or polysaccharide, the tally probe may comprise a carbohydrate-binding protein such as a lectin or a carbohydrate-binding antibody. The tally probe may be engineered to weaken its binding relative to the non-engineered version, e.g., to provide a binding and/or association with an analyte that is less thermodynamically stable and/or more transient than the non-engineered version. In addition to tally probes comprising antibodies, target analytes and tally probes that are proteins may be comprised, e.g., where one binding partner is the tally probe and the other binding partner is the analyte being measured. The use of aptamers binding any ligand, lectins binding glycosylated proteins, proteins or other molecules binding lipids, etc. are comprised. The technology comprises the use of any binding pair with transient binding behavior suitable for detection as described herein.

As used herein, the term "tally probe release component" refers to an entity (e.g., molecule, biomolecule (e.g., enzyme)) that promotes the dissociation of a tally probe from a tally probe-analyte complex, e.g., after transfer of a label from the tally probe to an integrator probe. That is, the tally probe release component converts a tally probe associated with an analyte to a tally probe that is dissociated from the analyte or that rapidly dissociates from the analyte (e.g., with a dissociation rate constant 0.1 min⁻¹ to approximately 10 min⁻¹ (e.g., 10.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0 min⁻¹)) under the assay conditions (e.g., incubation temperature, probe concentrations, pH, and/or salt concentration). A first portion of the tally probe (e.g., a portion comprising a label) may remain associated with the integrator probe and a second portion of the tally probe may dissociate from the analyte. The tally probe release component may be an enzyme (e.g., an RNase H) that degrades a portion of the tally probe (e.g., a portion of a tally probe comprising RNA) when it is bound to an analyte (e.g., a DNA analyte).

As used herein, the term "click chemistry" or "click reaction" refers to a chemical reaction that has a desirable chemical yield, yields a physiologically stable product, and that exhibits a large thermodynamic driving force that favors a "spring-loaded" reaction that yields a single product. See, e.g., Huisgen (1961) "Centenary Lecture - 1,3-Dipolar Cycloadditions", Proceedings of the Chemical Society of London 357; Kolb, Finn, Sharpless (2001) "Click Chemistry: Diverse Chemical Function from a Few Good Reactions", Angewandte Chemie International Edition 40(11): 2004-2021. Click chemistry may comprise Diels-Alder reactions, thiol-yne reactions, azide-alkyne reactions, tetrazine-trans-cyclooctene reactions, a combination thereof, or other reactions that form a single physiologically stable product (e.g., a covalent bond) with a desirable chemical yield and large thermodynamic driving force (e.g., in a "spring-loaded" reaction). For example, a foundational click reaction is the reaction of an alkyne with an azide group (e.g., N₃, e.g., N=N=N) in a copper (I)-catalyzed azide-alkyne cycloaddition ("CuAAC") reaction to form two new covalent bonds between azide nitrogens and alkyl carbons. The covalent bonds form a chemical link (e.g., comprising a five-membered triazole ring) between a first component and a second component that comprised the azide and the alkyne moieties before linkage. The reaction may be performed in a milieu comprising a copper-based catalyst such as Cu/Cu(OAc)₂, a tertiary amine such as tris-(benzyltriazolylmethyl)amine (TBTA), and/or tetrahydrofuran and acetonitrile (THF/MeCN). A click reaction may be a copper-free click reaction, e.g., a click reaction between a tetrazine moiety (e.g., a six-membered aromatic ring comprising four nitrogen atoms (C₂H₂N₄), e.g., 1,2,3,4-tetrazine, 1,2,3,5-tetrazine, or 1,2,4,5-tetrazine) and a trans-cyclooctene moiety (e.g., (CH2)₆(CH)₂). See, e.g., Jewett and Bertozzi (2010) "Cu-free click cycloaddition reactions in chemical biology" Chem Soc Rev 39: 1272-79; Freidel (2016) "Chemical tags for site-specific fluorescent labeling of biomolecules" Amino Acids 48: 1357-72; and Kim and Koo (2019) "Biomedical applications of copper-free click chemistry: in vitro, in vivo, and ex vivo" Chem Sci 10: 7835-51. As used herein, the two moieties that react in a click reaction to form a product are called a "first click reactant" and a "second click reactant". As used herein the "first click reactant" and the "second click reactant" form a "click product" in a click reaction. A "click catalyst" may promote the formation of a click product from the first click reactant and the second click reactant (e.g., by lowering the activation energy of the click reaction).

### Description

Although the disclosure herein refers to certain illustrated examples, it is to be understood that these examples are presented by way of example and not by way of limitation of the disclosed technology.

### Compositions for analyte detection

The technology disclosed herein provides compositions for detecting an analyte using an integrator probe and a tally probe. The integrator probe (I) binds stably (e.g., thermodynamically stable) to an analyte (A) and is capable of irreversibly and/or substantially irreversibly and/or effectively irreversibly binding (e.g., covalently or non-covalently) multiple copies of a label moiety (L). The tally probe (T) binds transiently to the analyte (A) and comprises a label moiety (L). Upon binding of the tally probe (T) to the analyte (A), the tally probe (T) irreversibly or substantially irreversibly or effectively irreversibly transfers a label moiety (L) to an integrator probe (I) (e.g., a proximal copy of the integrator probe (I)). That is, a tally probe (T) bound to the analyte (A) transfers a label moiety (L) to the integrator probe (I) bound to the same analyte molecule (A) with a high probability. The integrator probe (I) and the tally probe (T) are incubated with a composition comprising an analyte (A) for a sufficient length of time (e.g., an incubation time) to provide multiple cycles of tally probe (T) binding to each analyte (A) molecule present, thus producing one or more copies of the integrator probe (I) that is/are labeled with multiple copies of the label moiety (L). See, e.g., FIG. 1.

Thus, an integrator probe irreversibly binds multiple (e.g., at least 2) label moieties with high probability when (e.g., if and only if) the integrator probe is bound to the analyte (FIG. 1). In the absence of analyte (A), or in the presence of a spurious analyte (A*), the much lower bulk concentrations of integrator probe and tally probe provide that transfer of a label moiety to an integrator probe is an exceedingly rare event, and the vast majority of integrator probes comprise a small number of label moieties (e.g., 0 or 1) (FIG. 1). In other words, the presence of the analyte greatly accelerates the generation of multiply labeled integrator probes, which are generated only very slowly in the absence of the analyte. The analyte is detected by the presence of one or more copies of the integrator probe bearing at least N (e.g., at least 2) copies of the label. Detection is accomplished by any method that can distinguish between integrator probes comprising multiple label moieties from integrator probes comprising no or fewer label moieties.

As shown in FIG. 1, An integrator probe I binds stably to analyte A and repeated binding of tally probe T to the same copy of A triggers the transfer of a label L (star) from T to I. This transfer of the label is rapid due to the high local effective concentrations of I and T caused by binding to the same copy of A. Initially, I comprises no labels from the binding of T, and is designated I₀. After multiple cycles of T binding, I is modified by N ≥ 2 labels, and is designated I_{N}. I thus retains a memory of how many cycles of T binding have occurred to the associated copy of A. In the absence of analyte A (FIG. 1, Case 2), transfer of labels from T to I is slow because T and I are not colocalized (e.g., there is no locally high concentration of T and I and thus no enhancement of label transfer T to I). In the presence of a spurious analyte A* (FIG. 1, Case 3), binding of T and/or I to A* is weak, and the equilibrium favors states in which the two probes (I and T) are not simultaneously bound to the same copy of A*. The transfer of labels from T to I is thus much slower in the presence of A* than in the presence of A. The presence of multiple (e.g., more than 2) labels on a given copy of I thus provides a specific signature indicating the presence of A in the mixture.

An example of the technology for detecting a nucleic acid (e.g., DNA) analyte is shown in FIG. 2. In this example, each integrator probe comprises multiple tetrazine moieties (Tz); each tally probe comprises a region of RNA nucleotides ("RNA toehold") and one trans-cyclooctene moiety. A click chemistry reaction (e.g., a copper-free click chemistry reaction) between a tetrazine moiety (Tz) and a trans-cyclooctene moiety (TCO) is used to form a covalent bond between an integrator probe I and a tally probe T (e.g., comprising a label L). Hybridization mediates the interaction between tally T and analyte A. The interaction is rendered transient by the presence of RNase H, which degrades the portion of T that contains RNA nucleotides complementary to the analyte sequence. After multiple cycles of tally probe binding, click reaction, and degradation, multiple copies of T (which are partly degraded by RNase H) are covalently bound to each integrator probe. The analyte is detected by detecting integrator probe comprising multiple (e.g., more than 2) partially degraded tally probe molecules (e.g., comprising a label). For example, a multiply-bound integrator probe can be determined by denaturing polyacrylamide gel electrophoresis (PAGE) or by total internal reflection fluorescence (TIRF) microscopy (FIG. 2). This technology can be used with a relatively long or short interaction between the tally probe and the analyte and can be adapted for detection of other types of analytes, such as proteins (FIG. 2).

The tally probe may be a nucleic acid or an aptamer. The tally probe may be a low-affinity antibody, antibody fragment, or nanobody. The tally probe may be a DNA-binding protein, RNA-binding protein, or a DNA-binding ribonucleoprotein complex. The tally probe may comprise multiple types of molecules or moieties as described herein (e.g., nucleic acid and protein).

The integrator probe may be a high-affinity antibody, antibody fragment, or nanobody. The integrator probe may be a nucleic acid. The integrator probe may comprise multiple types of molecules or moieties as described herein (e.g., nucleic acid and protein). The integrator probe may comprise a separate phase of matter from the bulk mixture, such as a colloidal nanoparticle or a distinct phase of a liquid-in-liquid emulsion or other multi-phase system.

### Analytes

The technology is not limited in the analyte that is detected, quantified, identified, or otherwise characterized (e.g., presence, absence, amount, concentration, state). The term "analyte" as used herein is a broad term and is used in its ordinary sense, including, without limitation, to refer to a substance or chemical constituent in a sample such as a biological sample (e.g., a biofluid such as, e.g., blood, interstitial fluid, cerebral spinal fluid, lymph fluid or urine) that can be analyzed. Analytes can include naturally occurring substances, artificial substances, metabolites, and/or reaction products. The analyte may comprise a salt, sugars, protein, fat, vitamin, or a hormone. The analyte may be naturally present in a biological sample (e.g., is "endogenous"); for example, the analyte is a metabolic product, a hormone, an antigen, an antibody, and the like. Alternatively, the analyte may be introduced into a biological organism (e.g., is "exogenous), for example, a drug, drug metabolite, a drug precursor (e.g., prodrug), a contrast agent for imaging, a radioisotope, a chemical agent, etc. The metabolic products of drugs and pharmaceutical compositions are also contemplated analytes.

The analyte may be a polypeptide, a nucleic acid, a small molecule, a lipid, a carbohydrate, a polysaccharide, a fatty acid, a phospholipid, a glycolipid, a sphingolipid, an organic molecule, an inorganic molecule, a cofactor, a pharmaceutical, a bioactive agent, a cell, a tissue, an organism, etc. The analyte may comprise a polypeptide, a nucleic acid, a small molecule, a lipid, a carbohydrate, a polysaccharide, a fatty acid, a phospholipid, a glycolipid, a sphingolipid, an organic molecule, an inorganic molecule, a cofactor, a pharmaceutical, a bioactive agent, a cell, a tissue, an organism, etc. The analyte may comprise a combination of one or more of a polypeptide, a nucleic acid, a small molecule, a lipid, a carbohydrate, a polysaccharide, a fatty acid, a phospholipid, a glycolipid, a sphingolipid, an organic molecule, an inorganic molecule, a cofactor, a pharmaceutical, a bioactive agent, a cell, a tissue, an organism, etc.

The analyte may be a nucleic acid as described herein. In the invention the analyte is a nucleic acid. The analyte may be a nucleic acid that comprises a mutation (e.g., a single nucleotide polymorphism, a deletion, an insertion, a rearrangement, a fusion, etc.) The analyte may be a protein as described herein. The analyte may be a protein that comprises an amino acid substitution.

The analyte may be part of a multimolecular complex, e.g., a multiprotein complex, a nucleic acid/protein complex, a molecular machine, an organelle (e.g., a cell-free mitochondrion, e.g., in plasma; a plastid; golgi, endoplasmic reticulum, vacuole, peroxisome, lysosome, and/or nucleus), cell, virus particle, tissue, organism, or any macromolecular complex or structure or other entity that is amenable to analysis by the technology described herein (e.g., a ribosome, spliceosome, vault, proteasome, DNA polymerase III holoenzyme, RNA polymerase II holoenzyme, symmetric viral capsids, GroEL / GroES; membrane protein complexes: photosystem I, ATP synthase, nucleosome, centriole and microtubule-organizing center (MTOC), cytoskeleton, flagellum, nucleolus, stress granule, germ cell granule, or neuronal transport granule). For example, a multimolecular complex comprises an analyte and the technology finds use in characterizing, identifying, quantifying, and/or detecting one or more molecules (analytes) associated with (e.g., that is a component of) the multimolecular complex. An extracellular vesicle may be isolated and the technology may find use in characterizing, identifying, quantifying, and/or detecting one or more molecules (analytes) associated with the vesicle. The technology may find use in characterizing, identifying, quantifying, and/or detecting a protein (e.g., a surface protein) and/or an analytes present inside the vesicle, e.g., a protein, nucleic acid, or other analyte described herein. The vesicle may be fixed and permeabilized prior to analysis.

The interaction between the analyte and the tally probe may be distinguishably influenced by a covalent modification of the analyte. For example, the analyte may be a polypeptide comprising a post-translational modification, e.g., a protein or a peptide comprising a post-translational modification. A post-translational modification of a polypeptide may affect the association of a tally probe with the analyte, e.g., the integrator probe signal is a function of the presence or absence of the post-translational modification on the polypeptide. The interaction between the analyte comprising a post-translational modification and the tally probe may be mediated by a chemical affinity tag, e.g., a chemical affinity tag comprising a nucleic acid. For example, the analyte may be a nucleic acid comprising an epigenetic modification, e.g., a nucleic acid comprising a methylated base. A modification of a nucleic acid may affect the binding of a tally probe with the analyte, e.g., the integrator probe signal is a function of the presence or absence of the modification on the nucleic acid. The analyte may be a nucleic acid comprising a covalent modification to a nucleobase, a ribose, or a deoxyribose moiety of the target analyte.

The analyte may be subjected to thermal denaturation in the presence of a carrier prior to providing an integrator probe and/or a tally probe. The analyte may be subjected to chemical denaturation in the presence of a carrier prior to providing an integrator probe and/or a tally probe, e.g., the analyte is denatured with a denaturant such as urea, formamide, guanidinium chloride, high ionic strength, low ionic strength, high pH, low pH, or sodium dodecyl sulfate (SDS).

As used herein "detect an analyte" or "detect a substance" will be understood to encompass direct detection of the analyte itself or indirect detection of the analyte by detecting its by-product(s).

### Fluorescent labels and fluorescent moieties

The technology may comprise use of a fluorescent moiety (e.g., a fluorogenic dye, also referred to as a "fluorophore" or a "fluor"). For example, an integrator probe comprises one or more fluorescent labels (e.g., comprising a fluorescent moiety). A tally probe may comprise a fluorescent label (e.g., comprising a fluorescent moiety). A wide variety of fluorescent moieties is known in the art and methods are known for linking a fluorescent moiety to a molecule (e.g., an integrator probe and/or a tally probe).

Examples of compounds that may be used as the fluorescent moiety include but are not limited to xanthene, anthracene, cyanine, porphyrin, and coumarin dyes. Examples of xanthene dyes that find use with the present technology include but are not limited to fluorescein, 6-carboxyfluorescein (6-FAM), 5-carboxyfluorescein (5-FAM), 5- or 6-carboxy-4, 7, 2', 7'- tetrachlorofluorescein (TET), 5- or 6-carboxy-2', 4, 4', 5', 7, 7'-hexachlorofluorescein (HEX), 5' or 6'-carboxy-4',5'-dichloro-2,'7'-dimethoxyfluorescein (JOE), 5-carboxy-2', 4', 5', 7'-tetrachlorofluorescein (ZOE), rhodol, rhodamine, tetramethylrhodamine (TAMRA), 4, 7-dichlorotetramethyl rhodamine (DTAMRA), rhodamine X (ROX), and Texas Red. Examples of cyanine dyes that may find use with the present technology include but are not limited to Cy 3, Cy 3B, Cy 3.5, Cy 5, Cy 5.5, Cy 7, and Cy 7.5. Other fluorescent moieties and/or dyes that find use with the present technology include but are not limited to energy transfer dyes, composite dyes, and other aromatic compounds that give fluorescent signals. The fluorescent moiety may comprise a quantum dot.

The fluorescent moiety may comprise a fluorescent protein (e.g., a green fluorescent protein (GFP), a modified derivative of GFP (e.g., a GFP comprising S65T, an enhanced GFP (e.g., comprising F64L)), or others known in the art such as, e.g., blue fluorescent protein (e.g., EBFP, EBFP2, Azurite, mKalama1), cyan fluorescent protein (e.g., ECFP, Cerulean, CyPet, mTurquoise2), and yellow fluorescent protein derivatives (e.g., YFP, Citrine, Venus, YPet). The fluorescent protein may be covalently or noncovalently bonded to one or more integrator and/or tally probes.

Fluorescent dyes include, without limitation, d-Rhodamine acceptor dyes including Cy 5, dichloro[R110], dichloro[R6G], dichloro[TAMRA], dichloro[ROX] or the like, fluorescein donor dyes including fluorescein, 6-FAM, 5-FAM, or the like; Acridine including Acridine orange, Acridine yellow, Proflavin, pH 7, or the like; Aromatic Hydrocarbons including 2-Methylbenzoxazole, Ethyl p-dimethylaminobenzoate, Phenol, Pyrrole, benzene, toluene, or the like; Arylmethine Dyes including Auramine O, Crystal violet, Crystal violet, glycerol, Malachite Green or the like; Coumarin dyes including 7-Methoxycoumarin-4-acetic acid, Coumarin 1, Coumarin 30, Coumarin 314, Coumarin 343, Coumarin 6 or the like; Cyanine Dyes including 1,1'-diethyl-2,2'-cyanine iodide, Cryptocyanine, Indocarbocyanine (C3) dye, Indodicarbocyanine (C5) dye, Indotricarbocyanine (C7) dye, Oxacarbocyanine (C3) dye, Oxadicarbocyanine (C5) dye, Oxatricarbocyanine (C7) dye, Pinacyanol iodide, Stains all, Thiacarbocyanine (C3) dye, ethanol, Thiacarbocyanine (C3) dye, n-propanol, Thiadicarbocyanine (C5) dye, Thiatricarbocyanine (C7) dye, or the like; Dipyrrin dyes including N,N'-Difluoroboryl-1,9-dimethyl-5-(4-iodophenyl)-dipyrrin, N,N'-Difluoroboryl-1,9-dimethyl-5-[(4-(2-trimethylsilylethynyl), N,N'-Difluoroboryl-1,9-dimethyl-5-phenydipyrrin, or the like; Merocyanines including 4-(dicyanomethylene)-2-methyl-6-(p-dimethylaminostyryl)-4H-pyran (DCM), acetonitrile, 4-(dicyanomethylene)-2-methyl-6-(p-dimethylaminostyryl)-4H-pyran (DCM), methanol, 4-Dimethylamino-4'-nitrostilbene, Merocyanine 540, or the like; Miscellaneous Dyes including 4',6-Diamidino-2-phenylindole (DAPI), dimethylsulfoxide, 7-Benzylamino-4-nitrobenz-2-oxa-1,3-diazole, Dansyl glycine, Dansyl glycine, dioxane, Hoechst 33258, DMF, Hoechst 33258, Lucifer yellow CH, Piroxicam, Quinine sulfate, Quinine sulfate, Squarylium dye III, or the like; Oligophenylenes including 2,5-Diphenyloxazole (PPO), Biphenyl, POPOP, p-Quaterphenyl, p-Terphenyl, or the like; Oxazines including Cresyl violet perchlorate, Nile Blue, methanol, Nile Red, ethanol, Oxazine 1, Oxazine 170, or the like; Polycyclic Aromatic Hydrocarbons including 9,10-Bis(phenylethynyl)anthracene, 9,10-Diphenylanthracene, Anthracene, Naphthalene, Perylene, Pyrene, or the like; polyene/polyynes including 1,2-diphenylacetylene, 1,4-diphenylbutadiene, 1,4-diphenylbutadiyne, 1,6-Diphenylhexatriene, Beta-carotene, Stilbene, or the like; Redox-active Chromophores including Anthraquinone, Azobenzene, Benzoquinone, Ferrocene, Riboflavin, Tris(2,2'-bipyridypruthenium(II), Tetrapyrrole, Bilirubin, Chlorophyll a, diethyl ether, Chlorophyll a, methanol, Chlorophyll b, Diprotonated-tetraphenylporphyrin, Hematin, Magnesium octaethylporphyrin, Magnesium octaethylporphyrin (MgOEP), Magnesium phthalocyanine (MgPc), PrOH, Magnesium phthalocyanine (MgPc), pyridine, Magnesium tetramesitylporphyrin (MgTMP), Magnesium tetraphenylporphyrin (MgTPP), Octaethylporphyrin, Phthalocyanine (Pc), Porphin, ROX, TAMRA, Tetra-t-butylazaporphine, Tetra-t-butylnaphthalocyanine, Tetrakis(2,6-dichlorophenyl)porphyrin, Tetrakis(o-aminophenyl)porphyrin, Tetramesitylporphyrin (TMP), Tetraphenylporphyrin (TPP), Vitamin B12, Zinc octaethylporphyrin (ZnOEP), Zinc phthalocyanine (ZnPc), pyridine, Zinc tetramesitylporphyrin (ZnTMP), Zinc tetramesitylporphyrin radical cation, Zinc tetraphenylporphyrin (ZnTPP), or the like; Xanthenes including Eosin Y, Fluorescein, basic ethanol, Fluorescein, ethanol, Rhodamine 123, Rhodamine 6G, Rhodamine B, Rose bengal, Sulforhodamine 101, or the like; or mixtures or combination thereof or synthetic derivatives thereof.

Several classes of fluorogenic dyes and specific compounds are known that are appropriate for the technology: xanthene derivatives such as fluorescein, rhodamine, Oregon green, eosin, and Texas red; cyanine derivatives such as cyanine, indocarbocyanine, oxacarbocyanine, thiacarbocyanine, and merocyanine; naphthalene derivatives (dansyl and prodan derivatives); coumarin derivatives; oxadiazole derivatives such as pyridyloxazole, nitrobenzoxadiazole, and benzoxadiazole; pyrene derivatives such as cascade blue; oxazine derivatives such as Nile red, Nile blue, cresyl violet, and oxazine 170; acridine derivatives such as proflavin, acridine orange, and acridine yellow; arylmethine derivatives such as auramine, crystal violet, and malachite green; and tetrapyrrole derivatives such as porphin, phtalocyanine, bilirubin. The fluorescent moiety may be a dye that is xanthene, fluorescein, rhodamine, BODIPY, cyanine, coumarin, pyrene, phthalocyanine, phycobiliprotein, ALEXA FLUOR^{®} 350, ALEXA FLUOR^{®} 405, ALEXA FLUOR^{®} 430, ALEXA FLUOR^{®} 488, ALEXA FLUOR^{®} 514, ALEXA FLUOR^{®} 532, ALEXA FLUOR^{®} 546, ALEXA FLUOR^{®} 555, ALEXA FLUOR^{®} 568, ALEXA FLUOR^{®} 568, ALEXA FLUOR^{®} 594, ALEXA FLUOR^{®} 610, ALEXA FLUOR^{®} 633, ALEXA FLUOR^{®} 647, ALEXA FLUOR^{®} 660, ALEXA FLUOR^{®} 680, ALEXA FLUOR^{®} 700, ALEXA FLUOR^{®} 750, or a squaraine dye. The label may be a fluorescently detectable moiety as described in, e.g., Haugland (September 2005) MOLECULAR PROBES HANDBOOK OF FLUORESCENT PROBES AND RESEARCH CHEMICALS (10th ed.).

The label (e.g., a fluorescently detectable label) may be one available from ATTO-TEC GmbH (Am Eichenhang 50, 57076 Siegen, Germany), e.g., as described in U.S. Pat. Appl. Pub. Nos. 20110223677, 20110190486, 20110172420, 20060179585, and 20030003486; and in U.S. Pat. No. 7,935,822 (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO740).

One of ordinary skill in the art will recognize that dyes having emission maxima outside these ranges may be used as well. In some cases, dyes ranging between 500 nm to 700 nm have the advantage of being in the visible spectrum and can be detected using existing photomultiplier tubes. The broad range of available dyes allows selection of dye sets that have emission wavelengths that are spread across the detection range. Detection systems capable of distinguishing many dyes are known in the art.

### Label transfer

The technology comprises transfer (e.g., irreversible transfer (e.g., substantially irreversible transfer and/or effectively irreversible transfer)) of labels from a plurality of tally probes to an integrator probe. The technology is not limited in the compositions, molecules, linkers, non-covalent and/or covalent binding, chemical reactions, etc. that provide transfer of a label from a tally probe to an integrator probe when the tally probe and integrator probe are bound to an analyte molecule. The technology provides a tally probe comprising a label that is stably associated with the tally probe until the tally probe and an integrator probe are bound to an analyte. Upon association of the tally probe and integrator probe to an analyte, the label is transferred to the integrator probe and is stably associated with the integrator probe. Accordingly, the technology may provide: a tally probe comprising a label; and an integrator probe, wherein the affinity of the label for unbound tally probe is higher than the affinity of the label for any other molecule in the composition comprising the tally probe and the affinity of the label for the tally probe bound to an analyte is less than the affinity of the label for an integrator probe bound to the same analyte to which is bound the tally probe.

A tally probe may comprise a covalently linked label and a portion comprising RNA nucleotides that are complementary to a DNA analyte, the label chemically reacts (e.g., by a click chemistry reaction) with an integrator probe bound to the same analyte to which is bound the tally probe, and an RNase H enzyme degrades the portion of the tally probe comprising the RNA nucleotides to provide for binding of a different tally probe comprising a covalently linked label and a portion comprising RNA nucleotides that are complementary to a DNA analyte to provide a second cycle of label transfer to the integrator probe. This is more narrowly defined in the invention (see appended claims).

An integrator probe may comprise one or more nucleic acid duplexes comprising a toehold sequence, and a tally probe comprises a nucleic acid that is transferred to the integrator probe by toehold-mediated strand displacement.

An integrator probe may comprise a plurality of labels, each of which is in a first state and is converted to a second state by a tally probe bound to the same analyte to which is bound the integrator probe. Conversion of a plurality of integrator labels from the first state to the second state provides a record of the binding of a plurality of tally probes to the analyte.

A tally probe may comprise an antibody that binds a label and an integrator probe may comprise a plurality of antibodies that each bind the label with a higher affinity than the antibody of the tally probe binds the label.

An integrator probe may comprise one or more enzymes that catalyze(s) the transfer of one or more labels to the integrator probe. A tally probe may comprise one or more enzymes that catalyze(s) the transfer of a label to the integrator probe.

A tally probe may interact with the analyte indirectly via a separate adaptor probe (e.g., nucleic acid probe, aptamer, or antibody) that is bound stably to the analyte. Upon binding the adaptor probe, the tally probe transfers a label to an adjacent integrator probe associated to the same molecule of analyte, then dissociates from the adaptor probe. Many labels may be transferred to the same integrator probe from several tally probes that sequentially bind the same analyte via the same adaptor probe.

Binding of a tally probe to an analyte associated with a separate phase of matter in the reaction mixture (e.g., a colloidal particle, a lipid nanoparticle, a micelle, or a separate phase of a liquid-liquid emulsion, coacervate, or aqueous two-phase system) may result in the transfer of a label to the separate phase of matter. The integrator comprises the separate phase of matter.

### Detection

The technology comprises detecting an integrator probe comprising a plurality of labels (e.g., at least 2 labels). The technology provides for the detection of target analytes, e.g., in the presence of similar analytes and background noise. The technology is not limited in the detector or other detection technology that finds use in detecting the integrator probe comprising a plurality of labels. The label may be a fluorescent label and detecting an integrator probe comprising a plurality of labels may comprise detecting a fluorescent signal indicating that an integrator probe comprises a plurality of labels (e.g., a fluorescent signal indicating the presence of an integrator probe comprising more than one fluorescent label). The intensity of the fluorescence signal may indicate the number of labels attached to an integrator probe. A threshold may be established (e.g., a cut-off value) for the fluorescence intensity that distinguishes between an integrator probe comprising no or one label and an integrator probe comprising two or more labels. A threshold may be established (e.g., a cut-off value) for the fluorescence intensity that distinguishes between an integrator probe indicating the absence of an analyte (e.g., comprising a number of labels less than a threshold cutoff value) and an integrator probe indicating the presence of an analyte (e.g., comprising a number of labels that is at least a threshold value). Experiments are conducted using controls to establish a threshold (e.g., a cut-off value) for the fluorescence intensity and/or number of labels that distinguishes between the absence of an analyte (e.g., an integrator probe comprising no or one label) and the presence of an analyte (e.g., an integrator probe comprising two or more labels).

A threshold number of labels may be established indicating the presence from the absence of the analyte. For instance, fewer than 2 labels (e.g., 0 or 1 label) indicates the absence of the analyte and 2 or more labels indicates the presence of the analyte. Fewer than 3 labels (e.g., 0, 1, or 2 labels) may indicate the absence of the analyte and 3 or more labels may indicate the presence of the analyte. Fewer than 4 labels (e.g., 0, 1, 2, or 3 labels) may indicate the absence of the analyte and 4 or more labels may indicate the presence of the analyte (see, e.g., Example 2 and FIG. 4). Fewer than 5 labels (e.g., 0, 1, 2, 3, or 4 labels) may indicate the absence of the analyte and 5 or more labels may indicate the presence of the analyte. Fewer than 6 labels may indicate the absence of the analyte and 6 or more labels may indicate the presence of the analyte. Fewer than 7 labels may indicate the absence of the analyte and 7 or more labels may indicate the presence of the analyte. Fewer than 8 labels may indicate the absence of the analyte and 8 or more labels may indicate the presence of the analyte. Fewer than 9 labels may indicate the absence of the analyte and 9 or more labels may indicate the presence of the analyte. Fewer than 10 labels may indicate the absence of the analyte and 10 or more labels may indicate the presence of the analyte. Thresholds may be set at a cut-off of more than 10 labels.

The appropriate threshold number of labels to detect the presence of the analyte may be determined by statistical analysis of an appropriate blank or control measurement (e.g., by setting the threshold as 2, 3, 4, or 5 standard deviations above the mean or median number of labels per integrator detected, measured, and/or recorded for the blank or control measurement). The appropriate threshold number of labels to detect the presence of the analyte may be determined by subjecting the blank or control measurement to statistical analysis with a Poisson distribution, binomial distribution, normal distribution, or other applicable statistical model to determine (e.g., predict) the threshold number of labels that indicates the presence of the analyte with a probability of false positive detection events below a certain tolerable level (e.g., a probability of 0.01, 0.001, 0.0001, 0.00001, or 0.000001 that the detection of an analyte is a false positive).

The label may change the mass, charge, density, or hydrodynamic radius of an integrator probe and detecting an integrator probe comprising a plurality of labels comprises detecting a change in mass, charge, density, or hydrodynamic radius of an integrator probe indicating that an integrator probe comprises a plurality of labels (e.g., an assay comprising use of gel electrophoresis, mass spectrometry, gradient ultracentrifugation, chromatography, electrochemical assay). Detecting an integrator probe comprising a plurality of labels may comprise detecting a change in mobility using an electrophoretic and/or chromatographic method. See, e.g., FIG. 2 and FIG. 3.

A single-molecule detection method may be used to detect an integrator probe comprising a plurality of labels. For example, signal originating from an integrator probe comprising a plurality of labels is distinguishable from an integrator probe comprising zero labels or one label and/or from a tally probe comprising a label and/or from free label in a composition. Detecting an integrator probe comprising a plurality of labels may comprise use of a technology such as, e.g., total internal reflection fluorescence (TIRF) or near-TIRF microscopy, zero-mode waveguides (ZMWs), light sheet microscopy, stimulated emission depletion (STED) microscopy, or confocal microscopy. The integrator probe may comprise a plurality fluorescent labels and the intensity of the fluorescence emission may be proportional to the number of fluorescent labels attached to the integrator probe. The intensity of the fluorescence emission from the integrator probe may be compared to a known value of the intensity of the fluorescence emission for an integrator probe comprising one label. Detection of fluorescence having an intensity greater than the value for an integrator probe comprising one label indicates that the integrator probe comprises a plurality of labels and that the analyte is present in the sample. The fluorescence of an integrator probe comprising a plurality of labels may be greater than a defined threshold indicating the presence of the analyte in the sample. The fluorescence intensity of an integrator probe comprising a plurality of labels may have a fluorescence intensity that is at least 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 or more standard deviations above the fluorescence intensity of an integrator probe comprising zero or one label or above a defined threshold indicating the presence of an analyte in a sample.

The number of labels bound to an integrator probe may be inferred by counting the number of drops in fluorescent intensity as the integrator probe is exposed to excitation light (e.g., by counting of photobleaching steps). An integrator probe comprising a plurality of labels may exhibit at least 2, 3, 4, 5, 6, 7, 8, 9. 10, 11, 12, 13, 14, 15, 16, 17, 18, 19, or 20 photobleaching steps, indicating the presence of an analyte in a sample. An integrator probe exhibiting a number of photobleaching steps greater than that observed for integrator probes in control experiments in the absence of the analyte may indicate the presence of an analyte in a sample.

Positive and/or negative control samples may be measured (e.g., a control sample known to comprise or not to comprise an analyte). If a fluorescent label is used, fluorescence detected in a negative control sample is "background fluorescence" or "background (fluorescence) intensity" or "baseline".

Data may be analyzed by algorithms that recognize patterns and regularities in data, e.g., using artificial intelligence, pattern recognition, machine learning, statistical inference, neural nets, etc. The analysis may comprise use of a frequentist analysis and the analysis may comprise use of a Bayesian analysis. Pattern recognition systems may be trained using known "training" data (e.g., using supervised learning) and algorithms may be used to discover previously unknown patterns (e.g., unsupervised learning). See, e.g., Duda, et al. (2001) Pattern classification (2nd edition), Wiley, New York; Bishop (2006) Pattern Recognition and machine Learning, Springer. Pattern recognition (e.g., using training sets, supervised learning, unsupervised learning, and analysis of unknown samples) may associate identified patterns with analytes such that particular patterns provide a "fingerprint" of particular analytes that find use in detection, quantification, and identification of analytes. The data may be treated using statistics to determine the probability of an analyte being present or absent in a sample.

### Samples

A sample (e.g., a biological sample and/or a biofluid) may comprise an analyte. The sample may also comprise a variety of other non-analyte components, such as nucleic acids, proteins, lipids, and metabolites. Analytes and samples comprising analytes can be obtained from any material (e.g., cellular material (live or dead), extracellular material, viral material, environmental samples (e.g., metagenomic samples), and/or synthetic material), obtained from an animal, plant, bacterium, archaeon, fungus, or any other organism. Biological samples for use in the present technology include viral particles or preparations thereof. Analytes and samples comprising analytes can be obtained directly from an organism or from a biological sample obtained from an organism, e.g., from blood, urine, cerebrospinal fluid, seminal fluid, saliva, sputum, stool, hair, sweat, tears, skin, and tissue. Exemplary samples include, but are not limited to, whole blood, lymphatic fluid, serum, plasma, buccal cells, sweat, tears, saliva, sputum, hair, skin, biopsy, cerebrospinal fluid (CSF), amniotic fluid, seminal fluid, vaginal excretions, serous fluid, synovial fluid, pericardial fluid, peritoneal fluid, pleural fluid, transudates, exudates, cystic fluid, bile, urine, gastric fluids, intestinal fluids, fecal samples, and swabs, aspirates (e.g., bone marrow, fine needle, etc.), washes (e.g., oral, nasopharyngeal, bronchial, bronchialalveolar, optic, rectal, intestinal, vaginal, epidermal, etc.), and/or other specimens.

Any tissue or body fluid specimen may be used as a source for analytes and samples comprising analytes for use in the technology, including forensic specimens, archived specimens, preserved specimens, and/or specimens stored for long periods of time, e.g., fresh-frozen, methanol/acetic acid fixed, or formalin-fixed paraffin embedded (FFPE) specimens and samples. Analytes and samples comprising analytes can also be isolated from cultured cells, such as a primary cell culture or a cell line. The cells or tissues from which analytes and samples comprising analytes are obtained can be infected with a virus or other intracellular pathogen.

Nucleic acid molecules can be obtained, e.g., by extraction from a biological sample, e.g., by a variety of techniques such as those described by Maniatis, et al. (1982) Molecular Cloning: A Laboratory Manual, Cold Spring Harbor, N.Y. (see, e.g., pp. 280-281). A sample can comprise RNA extracted from a biological specimen, a cDNA library, viral nucleic acid, or genomic DNA. A sample may also be DNA isolated from a non-cellular origin, e.g. amplified/isolated DNA that has been stored in a freezer.

The technology may be used to identify an analyte in situ. In particular, the technology provides for the identification of an analyte directly in a tissue, cell, etc. (e.g., after permeabilizing the tissue, cell, etc.) without extracting the analyte from the tissue, cell, etc. The technology may be related to in situ detection, the technology may be applied in vivo, ex vivo, and/or in vitro. The sample may be a crude sample, a minimally treated cell lysates, or a biofluid lysate. The analyte may be detected in a crude lysates without nucleic acid purification.

### Methods

Provided herein is a method of identifying an analyte using an integrator probe and a tally probe as described herein. For example, methods comprise obtaining or providing a sample (e.g., a biological sample (e.g., a biofluid)), e.g., a sample comprising an analyte and/or suspected to comprise an analyte. The sample may be obtained from and/or provided by a patient in need of testing for the presence, absence, and/or quantity of an analyte. Testing a sample provided by and/or obtained from a patient for the presence, absence, and/or quantity of an analyte may indicate the status (e.g., the presence, absence, and/or quantity) of the analyte in the patient. A sample may be subjected to preliminary processing designed to isolate or enrich the sample for the analyte. A variety of techniques known to those of ordinary skill in the art may be used for this purpose, including but not limited to centrifugation, immunocapture, cell lysis, filtration, chromatography, use of magnetic beads, and nucleic acid target capture.

Methods may comprise providing an integrator probe and providing a tally probe. Methods may comprise mixing a composition comprising an analyte with one or more compositions comprising an integrator probe and/or a tally probe. Methods may comprise contacting a composition comprising an analyte with an integrator probe and a plurality of tally probes to provide a reaction mixture and incubating the reaction mixture at an incubation temperature for an incubation time.

Providing a tally probe may comprise synthesizing a tally probe. Synthesizing a tally probe may comprise synthesizing the tally probe and attaching (e.g., non-covalently or covalently attaching) a label to the tally probe. Methods may comprise providing the label. The label may be provided by a commercial source and the label may be synthesized by a user of the methods provided herein. A labeled tally probe may be provided by a commercial source. Providing an integrator probe may comprise synthesizing an integrator probe.

Methods may comprise incubating a composition comprising a tally probe and an integrator probe (e.g., at a temperature that is an incubation temperature and/or for a length of time that is an incubation time). Methods may comprise incubating a composition comprising a tally probe and an integrator probe for a length of time at the incubation temperature so that multiple cycles of tally probe binding to an analyte, if present, occur to produce an integrator probe comprising multiple labels. Methods may comprise detecting an integrator probe comprising multiple labels. Methods may comprise quantifying the number of integrator probes comprising multiple labels and/or quantifying the number of labels (e.g., label moieties) transferred from a tally probe to one or more integrator probes. Methods may comprise quantifying an analyte in a sample by quantifying the number of integrator probes comprising multiple labels and/or quantifying the number of labels (e.g., label moieties) transferred from a tally probe to an integrator probe. Methods may comprise producing a standard curve by quantifying the number of integrator probes comprising multiple labels and/or quantifying the number of labels (e.g., label moieties) transferred from a tally probe to an integrator probe in one or more compositions comprising a known amount and/or concentration of analyte under known conditions (e.g., a known incubation temperature and/or a known incubation time). Methods may comprise providing a negative control and/or providing a positive control. Methods may comprise performing an assay method with a negative control and/or with a positive control, e.g., by providing composition comprising the negative control and/or positive control, a tally probe, and an integrator probe.

Methods may comprise detecting a signal from a label. Methods may comprise providing a source of electromagnetic radiation. Methods may comprise providing a detector of electromagnetic radiation. Methods may comprise irradiating a sample (e.g., using a source of electromagnetic radiation). Methods may comprise quantifying an intensity of electromagnetic radiation, e.g., emitted by a label, using a detector of electromagnetic radiation. Methods may comprise calculating a concentration or amount of an analyte in a sample using an intensity of a signal produced by a label (e.g., by one or more labeled integrator probes).

Methods may comprise providing environmental conditions (e.g., temperature, solution conditions (e.g., pH, buffering, salt, chemical activities)), catalysts, and/or reactive chemical components so that a label is transferred from a tally probe to an integrator probe when a tally probe and an integrator probe are bound to the same analyte. Furthermore, methods may comprise providing environmental conditions (e.g., temperature, solution conditions (e.g., pH, buffering, salt, chemical activities)), catalysts, and/or reactive chemical components so that a tally probe comprising a label binds to an analyte to which is bound an integrator probe, a label is transferred from the tally probe to the integrator probe, and the tally probe dissociates from the analyte. Methods may comprise providing an enzymatic activity that promotes dissociation of a tally probe from an analyte after label transfer.

### Kits

The technology relates to kits. For example, kits comprise a tally probe comprising a label and an integrator probe, e.g., in one or more compositions (e.g., a composition comprising the tally probe comprising a label and the integrator probe; a first composition comprising the tally probe comprising a label and a second composition comprising an integrator probe). Kits may comprise a positive control (e.g., a composition comprising a known concentration and/or amount of an analyte). Kits may comprise a negative control (e.g., a composition comprising no analyte or an undetectable amount and/or concentration of an analyte). Kits may comprise a buffer solution for preparing a composition comprising an analyte. Kits may comprise a buffer solution and a device for preparing a sample from a patient. Kits may comprise a vessel for containing a sample from a subject and in which an assay as described herein is performed.

### Systems

The technology relates to systems for detecting analytes. For example, the technology provides a system for quantifying one or more target analytes, wherein the system comprises an integrator probe and a tally probe comprising a label as described herein. Furthermore, some systems may comprise a detection component that records a signal from the integrator probe after incubation of the integrator probe and tally probe with a sample comprising an analyte, if present. For example, the detection component records a signal produced from the integrator probe, e.g., after interaction of the integrator probe and tally probe with an analyte. The detection component may record the intensity of a signal provided by an integrator probe comprising a plurality of labels.

Systems may comprise analytical processes (e.g., embodied in a set of instructions, e.g., encoded in software, that direct a microprocessor to perform the analytical processes) to process a signal (e.g., from an integrator probe comprising a plurality of labels) and to identify a sample as a sample comprising an analyte. Analytical processes may use the intensity of the signal produced by an integrator probe comprising a plurality of labels as input data. Systems may comprise an analyte. Systems may not be limited in the analyte that is detected. For example, the analyte is polypeptide, e.g., a protein or a peptide. The target analyte may be a nucleic acid. The target analyte may be a small molecule or other molecule or entity as described herein.

Some systems of the technology comprise components for the detection and quantification of an analyte. Some systems comprise a detection component that is a fluorescence microscope comprising an illumination configuration to excite labels of integrator probes. Some systems comprise a fluorescence detector, e.g., a detector comprising an intensified charge coupled device (ICCD), an electron-multiplying charge coupled device (EM-CCD), a complementary metal-oxide-semiconductor (CMOS), a photomultiplier tube (PMT), an avalanche photodiode (APD), and/or another detector capable of detecting fluorescence emission from single chromophores. Some particular systems comprise a component configured for lens-free imaging, e.g., a lens-free microscope, e.g., a detection and/or imaging component for directly imaging on a detector (e.g., a CMOS) without using a lens.

Some systems comprise a computer and software encoding instructions for the computer to perform, e.g., to control data acquisition and/or analytical processes for processing data.

Some systems comprise optics, such as lenses, mirrors, dichroic mirrors, optical filters, etc., e.g., to detect fluorescence selectively within a specific range of wavelengths or multiple ranges of wavelengths.

For example, computer-based analysis software is used to translate the raw data generated by the detection assay (e.g., the presence, absence, or amount of one or more analytes) into data of predictive value for a clinician. The clinician can access the predictive data using any suitable means.

Some systems comprise a computer system upon which the present technology may be implemented. A computer system may include a bus or other communication mechanism for communicating information and a processor coupled with the bus for processing information. The computer system may include a memory, which can be a random access memory (RAM) or other dynamic storage device, coupled to the bus, and instructions to be executed by the processor. Memory also can be used for storing temporary variables or other intermediate information during execution of instructions to be executed by the processor. The computer system can further include a read only memory (ROM) or other static storage device coupled to the bus for storing static information and instructions for the processor. A storage device, such as a magnetic disk or optical disk, can be provided and coupled to the bus for storing information and instructions.

The computer system may be coupled via the bus to a display, such as a cathode ray tube (CRT) or a liquid crystal display (LCD), for displaying information to a computer user. An input device, including alphanumeric and other keys, can be coupled to the bus for communicating information and command selections to the processor. Another type of user input device is a cursor control, such as a mouse, a trackball, or cursor direction keys for communicating direction information and command selections to the processor and for controlling cursor movement on the display. This input device typically has two degrees of freedom in two axes, a first axis (e.g., x) and a second axis (e.g., y), that allows the device to specify positions in a plane.

A computer system can perform the present technology. Consistent with certain implementations of the present technology, results can be provided by the computer system in response to the processor executing one or more sequences of one or more instructions contained in the memory. Such instructions can be read into the memory from another computer-readable medium, such as a storage device. Execution of the sequences of instructions contained in the memory can cause the processor to perform the methods described herein. Alternatively, hard-wired circuitry can be used in place of or in combination with software instructions to implement the present teachings. Thus, implementations of the present technology are not limited to any specific combination of hardware circuitry and software.

The term "computer-readable medium" as used herein refers to any medium that participates in providing instructions to the processor for execution. Such a medium can take many forms, including but not limited to, non-volatile media, volatile media, and transmission media. Examples of non-volatile media can include, but are not limited to, optical or magnetic disks, such as a storage device. Examples of volatile media can include, but are not limited to, dynamic memory. Examples of transmission media can include, but are not limited to, coaxial cables, copper wire, and fiber optics, including the wires that comprise the bus.

Common forms of computer-readable media include, for example, a floppy disk, a flexible disk, hard disk, flash medium, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

Various forms of computer readable media can be involved in carrying one or more sequences of one or more instructions to the processor for execution. For example, the instructions can initially be carried on the magnetic disk of a remote computer. The remote computer can load the instructions into its dynamic memory and send the instructions over a network connection (e.g., a LAN, a WAN, the internet, a telephone line). A local computer system can receive the data and transmit it to the bus. The bus can carry the data to the memory, from which the processor retrieves and executes the instructions. The instructions received by the memory may optionally be stored on a storage device either before or after execution by the processor.

Instructions configured to be executed by a processor to perform a method may be stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

In accordance with such a computer system, functionalities for collecting, storing, and/or analyzing data (e.g., presence, absence, concentration of an analyte) may be further comprised. For example, a system that comprises a processor, a memory, and/or a database for, e.g., storing and executing instructions, analyzing label signals, signal intensities, and/or detection data, performing calculations using the data, transforming the data, and storing the data may be contemplated. An algorithm may apply a statistical model to the data.

Many diagnostics involve determining the presence of, or a nucleotide sequence of, one or more nucleic acids.

An equation comprising variables representing the presence, absence, concentration, amount, or sequence properties of one or more analytes may produce a value that finds use in making a diagnosis or assessing the presence or qualities of an analyte. As such, this value may be presented by a device, e.g., by an indicator related to the result (e.g., an LED, an icon on a display, a sound, or the like). A device may store the value, may transmit the value, or may use the value for additional calculations. An equation may comprise variables representing the presence, absence, concentration, amount, or properties of one or more analytes.

Thus, the present technology may provide the further benefit that a clinician, who is not likely to be trained in analytical assays, need not understand the raw data. The data are presented directly to the clinician in its most useful form. The clinician may be then able to utilize the information to optimize the care of a subject. The present technology contemplates any method capable of receiving, processing, and transmitting the information to and from laboratories conducting the assays, information providers, medical personal, and/or subjects. For example, a sample is obtained from a subject and submitted to a profiling service (e.g., a clinical lab at a medical facility, genomic profiling business, etc.), located in any part of the world (e.g., in a country different than the country where the subject resides or where the information is ultimately used) to generate raw data. Where the sample comprises a tissue or other biological sample, the subject may visit a medical center to have the sample obtained and sent to the profiling center or subjects may collect the sample themselves and directly send it to a profiling center. Where the sample comprises previously determined biological information, the information may be directly sent to the profiling service by the subject (e.g., an information card containing the information may be scanned by a computer and the data transmitted to a computer of the profiling center using electronic communication systems). Once received by the profiling service, the sample is processed, and a profile is produced that is specific for the diagnostic or prognostic information desired for the subject. The profile data are then prepared in a format suitable for interpretation by a treating clinician. For example, rather than providing raw data, the prepared format may represent a diagnosis or risk assessment for the subject, along with recommendations for particular treatment options. The data may be displayed to the clinician by any suitable method. For example, the profiling service may generate a report that can be printed for the clinician (e.g., at the point of care) or displayed to the clinician on a computer monitor. The information may be first analyzed at the point of care or at a regional facility. The raw data are then sent to a central processing facility for further analysis and/or to convert the raw data to information useful for a clinician or patient. The central processing facility provides the advantage of privacy (all data are stored in a central facility with uniform security protocols), speed, and uniformity of data analysis. The central processing facility can then control the fate of the data following treatment of the subject. For example, using an electronic communication system, the central facility can provide data to the clinician, the subject, or researchers. The subject may be able to access the data using the electronic communication system. The subject may choose further intervention or counseling based on the results. The data may be used for research use. For example, the data may be used to further optimize the inclusion or elimination of markers as useful indicators of a particular condition associated with the disease.

### Uses

The technology is not limited in its use. For example, the technology finds use in research for detecting the presence of an analyte and/or for quantifying an analyte. The technology may find use in clinical medicine, e.g., in detecting an analyte that indicates that a patient has a disease, malady, and/or ailment.

The technology relates to the detection of a wide range of analytes. For example, the technology finds use in detecting a nucleic acid (e.g., a DNA or RNA). The technology may find use in detecting a nucleic acid comprising a particular target sequence. The technology may find use in detecting a nucleic acid comprising a particular mutation (e.g., a single nucleotide polymorphism, an insertion, a deletion, a missense mutation, a nonsense mutation, a genetic rearrangement, a gene fusion, etc.). The technology may find use in detection a polypeptide (e.g., a protein, a peptide). The technology may find use in detecting a polypeptide encoded by a nucleic acid comprising a mutation (e.g., a polypeptide comprising a substitution, a truncated polypeptide, a mutant or variant polypeptide).

The technology may find use in detecting post-translational modifications to polypeptides (e.g., phosphorylation, methylation, acetylation, glycosylation (e.g., O-linked glycosylation, N-linked glycosylation, ubiquitination, attachment of a functional group (e.g., myristoylation, palmitoylation, isoprenylation, prenylation, farnesylation, geranylation, geranylgeranylation, glypiation, glycosylphosphatidylinositol (GPI) anchor formation), hydroxylation, biotinylation, pegylation, oxidation, SUMOylation, disulfide bridge formation, disulfide bridge cleavage, proteolytic cleavage, amidation, sulfation, pyrrolidone carboxylic acid formation. The technology may find use in the detection of the loss of these features, e.g., dephosporylation, demethylation, deacetylation, deglycosylation, deamidation, dehydroxylation, deubiquitination, etc. The technology may find use in detecting epigenetic modifications to DNA or RNA (e.g., methylation (e.g., methylation of CpG sites), hydroxymethylation). The technology may find use in detecting the loss of these features, e.g., demethylation of DNA or RNA, etc. The technology may find use in detecting alterations in chromatin structure, nucleosome structure, histone modification, etc., and in detecting damage to nucleic acids. The technology may find use in detecting a lipid, carbohydrate, metabolite, and/or small molecule.

The technology may find use as a molecular diagnostic assay, e.g., to assay samples having small specimen volumes (e.g., a droplet of blood, e.g., for mail-in service). The technology may provide for the early detection of cancer or infectious disease using sensitive detection of very low-abundance analyte biomarkers. The technology may find use in molecular diagnostics to assay epigenetic modifications of protein biomarkers (e.g., post-translational modifications).

The technology may find use in characterizing multimolecular complexes (e.g., characterizing one or more components of a multimolecular complex), e.g., a multiprotein complex, a nucleic acid/protein complex, a molecular machine, an organelle (e.g., a cell-free mitochondrion, e.g., in plasma), cell, virus particle, organism, tissue, or any macromolecular structure or entity that is amenable to analysis by the technology described herein. For example, a multimolecular complex is isolated and the technology finds use in characterizing, identifying, quantifying, and/or detecting one or more molecules (analytes) associated with the multimolecular complex. An extracellular vesicle may be isolated and the technology finds use in characterizing, identifying, quantifying, and/or detecting one or more molecules (analytes) associated with the vesicle. The technology may find use in characterizing, identifying, quantifying, and/or detecting a protein (e.g., a surface protein) and/or an analyte present inside the vesicle, e.g., a protein, nucleic acid, or other analyte described herein. The vesicle may be fixed and permeabilized prior to analysis.

### EXAMPLES

### Example 1

During the development of the technology described herein, experiments were conducted to detect a point mutation in a nucleic acid using the integrator technology described herein. In the experiment, an integrator probe comprising one or more (e.g., 1, 2, or 3) trans-cyclooctene (TCO) moieties and a tally probe comprising methyltetrazine (mTz) were used to detect a cytosine-to-thymine point mutation in a nucleic acid encoding epidermal growth factor receptor (EGFR). This mutation produces a substitution of methionine for threonine at position 790 in the amino acid sequence of the EGFR protein (EGFR T790M).

In an exemplary experiment, the EGFR T790M nucleic acid ("MUT") was 41 bp in length. The tally probe comprised 10 consecutive nucleotides complementary to the portion of MUT containing the single-nucleotide mutation. Six nucleotides of the tally probe 10-nt complementary sequence were RNA nucleotides and provided a region that was targeted for degradation by RNase H upon binding of the tally probe to the MUT nucleic acid. A wild-type competitor probe was provided that comprised 10 DNA nucleotides that are complementary to the wild-type (WT) EGFR sequence. The competitor probe competed with the tally probe for binding to the wild-type sequence and suppressed nonspecific signal that could be generated by interaction of the tally probe with the wild-type nucleic acid. The analyte (MUT nucleic acid), integrator probe, tally probe, and competitor probe were incubated with RNase H in NEBuffer 3.1 (New England Biolabs) at room temperature for 1-30 minutes before quenching the click reaction with approximately 2 mM methyltetrazine. Reactions were run on a denaturing polyacrylamide gel and SYBR gold staining was used to visualize nucleic acids within the gel (FIG. 3).

As described above, the tally probe was designed to form 10 base pairs with the nucleic acid encoding EGFR T790M and a competitor probe was designed to form 10 base pairs with the wild-type nucleotide sequence to suppress binding of the tally probe to the wild-type nucleic acid. Accordingly, the experiment was designed so that the integrator TCO moieties reacted (e.g., by click chemistry reactions) with multiple copies of the tally probe only if the mutant sequence EGFR T790M was present and not if the wild-type sequence was present. Data collected during the experiment were consistent with this expectation. As shown in FIG. 3, low-mobility bands appeared in a denaturing polyacrylamide gel electrophoresis experiment upon incubation of the integrator probe, tally probe, wild-type competitor probe, and RNase H with the mutant nucleic acid (MUT). These low-mobility bands indicated the presence of the integrator probe conjugated to 2-3 copies of the tally probe. In contrast, there was no evidence of a multiply-modified integrator probe in the presence of only the wild-type sequence (FIG. 3), demonstrating the ability of the technology described herein to distinguish analytes, e.g., nucleic acids comprising sequences that differ by as little as a single nucleotide.

### Example 2

During the development of the technology provided herein, experiments were conducted in which the technology was used to detect an analyte DNA sequence encoding the EGFR T790M mutation using a single integrator molecule and single-molecule total internal reflection fluorescence (TIRF) microscopy. Samples comprised a target analyte that was a DNA comprising a nucleotide sequence encoding the EGFR T790M mutation (FIG. 4, bottom) or lacked the target analyte (negative control; FIG. 4, top). After incubation of the negative control with an integrator probe specific for the sequence encoding the EGFR mutation, a tally probe, and RNase H, the integrator probes typically comprised only 0, 1, or 2 fluorescent labels and were not observed to comprise more than 3 fluorescent labels (FIG. 4, top). In contrast, after incubation of a sample comprising the target analyte with the integrator probe, the tally probes, and RNase H, a significant subset of integrator probes comprised 4, 5, or 6 labels, indicating the presence of the target sequence in the mixture (FIG. 4, bottom). The number of labels associated with each integrator probe after incubation can be inferred by the number of drops in fluorescence (e.g., corresponding to photobleaching steps) observed for each surface-bound integrator probe as the sample is exposed to excitation light. In the particular system used in these experiments, the integrator probe was configured to bind to as many as six labels, which comprise a DNA sequence, and the labels were detected by sequence-specific hybridization of a fluorescently labeled DNA probe to the label. Furthermore, these data indicate that embodiments of the technology provided herein detect single integrator molecules (e.g., using TIRF microscopy) that were bound to single analyte molecules during the incubation period. As shown in FIG. 4, multiply labeled integrator probes appear as brighter puncta in the microscope image and can be detected by counting the number of photobleaching steps per integrator molecule.

## Claims

1. A composition for detecting a nucleic acid analyte in a sample, said composition comprising:
i) a nucleic acid integrator probe comprising
- multiple first click chemistry groups;
ii) a nucleic acid tally probe comprising a region of RNA nucleotides targeted for RNase degradation and capable of binding to said nucleic acid analyte, said nucleic acid tally probe comprising
- a label, and covalently linked to the label
- a second click chemistry group,
wherein
said first click chemistry groups and said second click chemistry group are complementary click chemistry groups,
both said nucleic acid integrator probe and said nucleic acid tally probe are capable of binding said nucleic acid analyte in proximity and becoming connected to each other via reaction of said complementary click chemistry groups, thereby associating said label, first associated with the nucleic acid tally probe, also with said nucleic acid integrator probe resulting in an integrator-tally conjugate,
wherein said detection involves addition of an RNase after addition of composition to the sample, in order to degrade said region of RNA nucleotides of said nucleic acid tally probe.

2. The composition of claim 1, wherein said multiple first click chemistry groups are tetrazine moieties and said second click chemistry group is a trans-cyclooctene moiety.

3. The composition of claim 2, wherein a copper-free click chemistry reaction between the tetrazine moiety and the trans-cyclooctene moiety is to be used to form a covalent bond between said nucleic acid integrator probe and said nucleic acid tally probe.

4. The composition of any of the preceding claims, wherein in said detection multiple copies of RNase degraded tally probes will have become conjugated to said nucleic acid integrator probe after multiple cycles of tally probe binding to the nucleic acid analyte, click reaction, and RNase degradation mediated release from said nucleic acid analyte.

5. The composition of any of the preceding claims, wherein in said detection said integrator-tally conjugate can be determined by denaturing polyacrylamide gel electrophoresis or by total internal reflection fluorescence microscopy.

6. The composition of claim 1, wherein said integrator probe is configured to bind more than one label.

## Patentansprüche

1. Zusammensetzung zum Nachweis eines Nukleinsäure-Analyt in einer Probe, wobei besagte Zusammensetzung umfasst:
i) eine Nukleinsäure-Integratorsonde, umfassend
- mehrere erste Click-Chemie-Gruppen;
ii) eine Nukleinsäure-Zählsonde, die einen Bereich von RNA-Nukleotiden umfasst, der für den RNase-Abbau vorgesehen ist und an besagten Nukleinsäure-Analyt binden kann, wobei besagte Nukleinsäure-Zählsonde umfasst
- einen Marker und kovalent an den Marker gebunden
- eine zweite Click-Chemie-Gruppe,
wobei
besagte erste Click-Chemie-Gruppen und besagte zweite Click-Chemie-Gruppe komplementäre Click-Chemie-Gruppen sind,
sowohl besagte Nukleinsäure-Integratorsonde als auch besagte Nukleinsäure-Zählsonde in der Lage sind, besagten Nukleinsäure-Analyt in der Nähe zu binden und über die Reaktion der komplementären Click-Chemie-Gruppen miteinander verbunden zu werden, wodurch besagter Marker, der zunächst an die Nukleinsäure-Zählsonde gebunden ist, auch an die Nukleinsäure-Integratersonde gebunden wird, was zu einem Integrator-Zähl-Konjugat führt,
wobei besagter Nachweis die Zugabe einer RNase nach Zugabe der Zusammensetzung zur Probe umfasst, um besagten Bereich der RNA-Nukleotide besagter Nukleinsäure-Zählsonde abzubauen.

2. Zusammensetzung nach Anspruch 1, wobei besagte mehrere ersten Click-Chemie-Gruppen Tetrazin-Reste sind und besagte zweite Click-Chemie-Gruppe ein trans-Cycloocten-Rest ist.

3. Zusammensetzung nach Anspruch 2, wobei eine kupferfreie Klick-Chemie-Reaktion zwischen dem Tetrazin-Rest und dem trans-Cycloocten-Rest verwendet wird, um eine kovalente Bindung zwischen besagter Nukleinsäureintegrator-Sonde und besagter Nukleinsäure-Zählsonde zu bilden.

4. Zusammensetzung nach einem der vorangehenden Ansprüche, wobei bei besagtem Nachweis mehrere Kopien von RNase-abgebauten Zählproben nach mehreren Zyklen der Bindung der Zählprobe an den Nukleinsäure-Analyt, der Klickreaktion und der durch RNase-Abbau vermittelten Freisetzung aus dem Nukleinsäure-Analyt an die Nukleinsäure-Integratorsonde konjugiert sind.

5. Zusammensetzung nach einem der vorangehenden Ansprüche, wobei bei besagtem Nachweis das Integrator-Zähl-Konjugat durch denaturierende Polyacrylamid-Gelelektrophorese oder durch Totalreflexionsfluoreszenzmikroskopie bestimmt werden kann.

6. Zusammensetzung nach Anspruch 1, wobei besagte Integratorsonde so konfiguriert ist, dass sie mehr als einen Marker bindet.

## Revendications

1. Composition pour détecter un analyte d'acide nucléique dans un échantillon, ladite composition comprenant :
i) une sonde intégratrice d'acides nucléiques comprenant
- plusieurs premiers groupes de chimie clic ;
ii) une sonde de comptage d'acides nucléiques comprenant une région de nucléotides d'ARN ciblée pour la dégradation de RNase et capable de se lier audit analyte d'acide nucléique, ladite sonde de comptage d'acides nucléiques comprenant
- un marqueur, et lié de manière covalente au marqueur
- un deuxième groupe de chimie clic,
dans laquelle
lesdits premiers groupes de chimie clic et ledit deuxième groupe de chimie clic sont des groupes de chimie clic complémentaires,
à la fois ladite sonde intégratrice d'acides nucléiques et ladite sonde de comptage d'acides nucléiques sont capables de se lier audit analyte d'acide nucléique à proximité et de se connecter l'une à l'autre par réaction desdits groupes de chimie clic complémentaires, associant ainsi ledit marqueur, d'abord associé à la sonde de comptage d'acides nucléiques, également à ladite sonde intégratrice d'acides nucléiques, ce qui donne un conjugué intégrateur-comptage, ladite détection impliquant l'addition d'une RNase après addition de la composition à l'échantillon, afin de dégrader ladite région de nucléotides d'ARN de ladite sonde de comptage d'acides nucléiques.

2. Composition selon la revendication 1, dans laquelle lesdits plusieurs premiers groupes de chimie clic sont des groupements tétrazine et ledit deuxième groupe de chimie clic est un groupement trans-cyclooctène.

3. Composition selon la revendication 2, dans laquelle une réaction de chimie clic sans cuivre entre le groupement tétrazine et le groupement trans-cyclooctène doit être utilisée pour former une liaison covalente entre ladite sonde intégratrice d'acides nucléiques et ladite sonde de comptage d'acides nucléiques.

4. Composition selon l'une quelconque des revendications précédentes, dans laquelle, dans ladite détection, de multiples copies de sondes de comptage dégradées par RNase seront devenues conjuguées à ladite sonde intégratrice d'acides nucléiques après plusieurs cycles de liaison de la sonde de comptage à l'analyte d'acide nucléique, de réaction de clic et de libération médiée par la dégradation par une RNase à partir dudit analyte d'acide nucléique.

5. Composition selon l'une quelconque des revendications précédentes, dans laquelle, dans ladite détection, ledit conjugué intégrateur-comptage peut être déterminé par électrophorèse sur gel de polyacrylamide dénaturant ou par microscopie à fluorescence à réflexion interne totale.

6. Composition selon la revendication 1, dans laquelle ladite sonde intégratrice est configurée pour se lier à plus d'un marqueur.
